# EUROPEAN PATENT APPLICATION

(11) **EP 4 822 011 A1**
(43) Date of publication of application: **14.10.2026**
(21) Application number: 24900727.9
(22) Date of filing: 09.12.2024
(51) Int. Cl.: C12N 1/00, C12N 15/12

(54) **METHOD FOR SEPARATING EXTRACELLULAR VESICLES, AND KIT FOR SEPARATING EXTRACELLULAR VESICLES**

(30) Priority: 08.12.2023 JP 2023207830
(71) Applicant: National Institute of Advanced Industrial Science and Technology, Chiyoda-ku Tokyo 100-8921 (JP)
(72) Inventor: TATENO, Hiroaki, Tsukuba-shi, Ibaraki 305-8560 (JP)
(74) Representative: Vossius & Partner Patentanwälte Rechtsanwälte mbB
(86) International application number: PCT/JP2024/043415
(87) International publication number: WO 2025/121435

(57) **Abstract**

The problem to be addressed is providing a method for separating extracellular vesicles and a kit for separating extracellular vesicles.

Provided is a separation method for separating extracellular vesicles in a sample, the method including contacting the extracellular vesicles in the sample with a C-type lectin and separating the extracellular vesicles bound to the C-type lectin from the C-type lectin. Also provided is a kit for separating extracellular vesicles from a sample including extracellular vesicles, the kit including a C-type lectin.

## Description

### TECHNICAL FIELD

The present disclosure relates to a method for separating extracellular vesicles and a kit for separating extracellular vesicles.

### BACKGROUND

Extracellular vesicles (EVs) are spherical membrane vesicles about 30-1000 nm in size that are composed of a lipid bilayer and secreted extracellularly from all cells.

Extracellular vesicles include therein bioinformation molecules such as DNA, RNA (mRNA, miRNA, ncRNA, etc.), proteins, and glycans, which mediate various physiological functions as transmitters.

The use of extracellular vesicles for the diagnosis of various diseases such as cancer by detecting encapsulated miRNA is progressing. Further, mesenchymal stem cell-derived extracellular vesicles have anti-inflammatory action, immunoregulatory action, tissue-repairing action, and the like, so the use thereof in therapeutic formulations is advancing. Development of methods of application such as use as a delivery system for drugs such as nucleic acid medicaments is ongoing.

Along with these advances, the development of methods for identifying and separating extracellular vesicles is also going forward. However, there are no established methods at the present stage, and many methods have been developed, proposed, and used.

Generally, ultracentrifugation methods are used when separating extracellular vesicles. However, there are the issues with these methods of it not being possible to process a large number of samples at once and the extracellular vesicles tending to be contaminated by impurities.

Furthermore, there are close subtypes of extracellular vesicles, and there is a need for methods of isolating only extracellular vesicles including a desired active component. Recently, methods have also been developed for separating Tim4, which binds to phosphatidylserine present on the surfaces of extracellular vesicles, with immobilized magnetic beads. However, even with these methods, extracellular vesicles that do not include the active component are intermixed.
Patent Document 1: JP 2021-012200 A
Patent Document 2: WO 2016/088689 A

### SUMMARY OF INVENTION

The present disclosure addresses the problem of providing a method for separating extracellular vesicles and a kit for separating extracellular vesicles.

The present disclosure includes the following aspects.
[1-1] A method for separating extracellular vesicles in a sample, including:
   contacting the extracellular vesicles in the sample with a C-type lectin; and
   separating the extracellular vesicles bound to the C-type lectin from the C-type lectin.
[1-2] The separation method described in [1-1], including:
   contacting the extracellular vesicles in the sample with the C-type lectin in the presence of a calcium ion; and
   separating the extracellular vesicles bound to the C-type lectin from the C-type lectin in the absence of the calcium ion.
[1-3] The separation method described in [1-1] or [1-2], wherein the C-type lectin is ASGR1, DCSIGN, and/or CLEC1B.
[1-4] The separation method described in any of [1-1] to [1-3], wherein the C-type lectin is ASGR1.
[2-1] A kit for separating extracellular vesicles in a sample, wherein the kit includes a C-type lectin.
[2-2] A kit for detecting extracellular vesicles including a C-type lectin and an antibody that specifically binds to the extracellular vesicles.
[2-3] The kit described in [2-1] or [2-2], wherein the C-type lectin is ASGR1, DCSIGN, and/or CLEC1B.
[2-4] The kit described in [2-3], wherein the C-type lectin is ASGR1.

According to the present disclosure, a method for separating extracellular vesicles and a kit for separating extracellular vesicles can be provided.

### BRIEF DESCRIPTION OF DRAWINGS

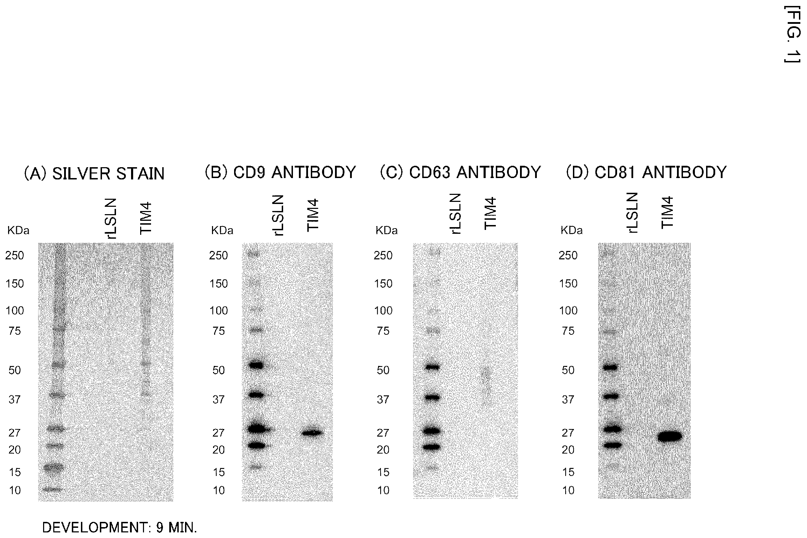

Fig. 1 is silver staining (A) as well as western blots using an anti-CD9 antibody (B), an anti-CD63 antibody (C), and an anti-CD81 antibody (D) of extracellular vesicles separated with a TIM4 method in Example 1.

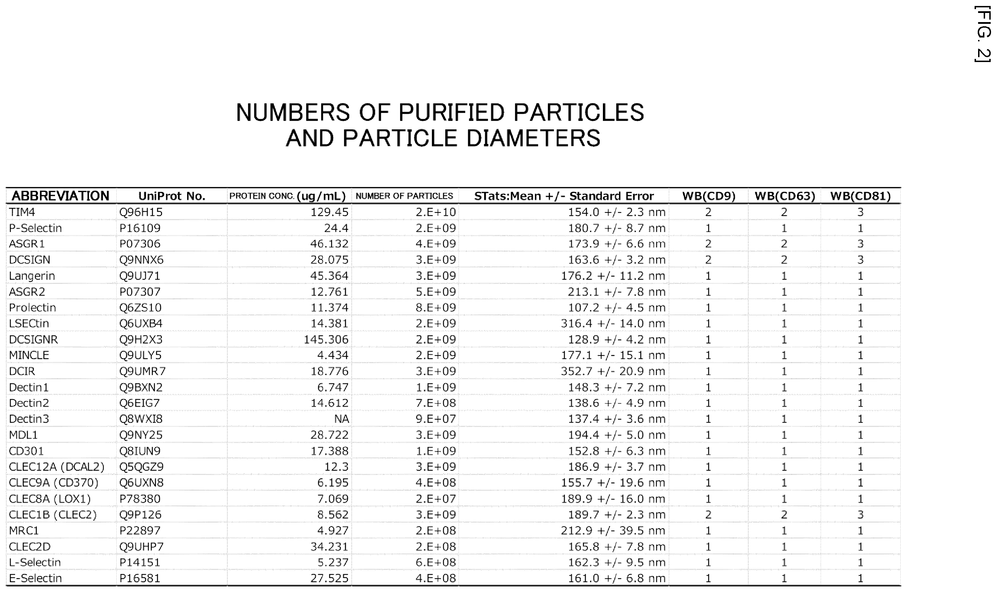

Fig. 2 shows the lectin used, the protein concentrations, the numbers of particles, and the particle diameters of extracellular vesicles (mean ± standard error) and the results of western blotting using an anti-CD9 antibody, an anti-CD63 antibody, and an anti-CD81 antibody in the separation of extracellular vesicles using human C-type lectins in Example 2.

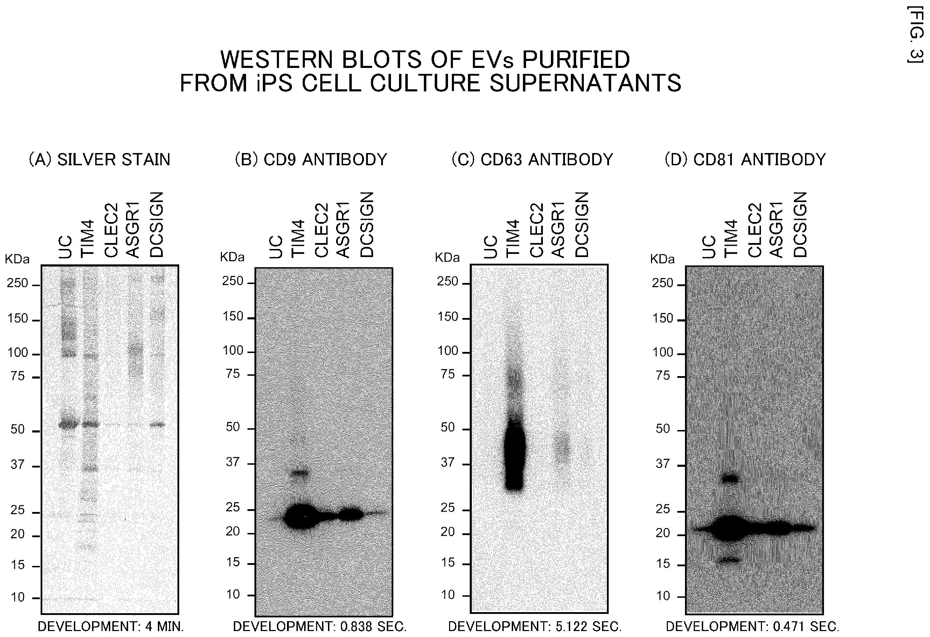

Fig. 3 is silver staining (A) as well as western blots using an anti-CD9 antibody (B), an anti-CD63 antibody (C), and an anti-CD81 antibody (D) of extracellular vesicles separated using human C-type lectins in Example 3.

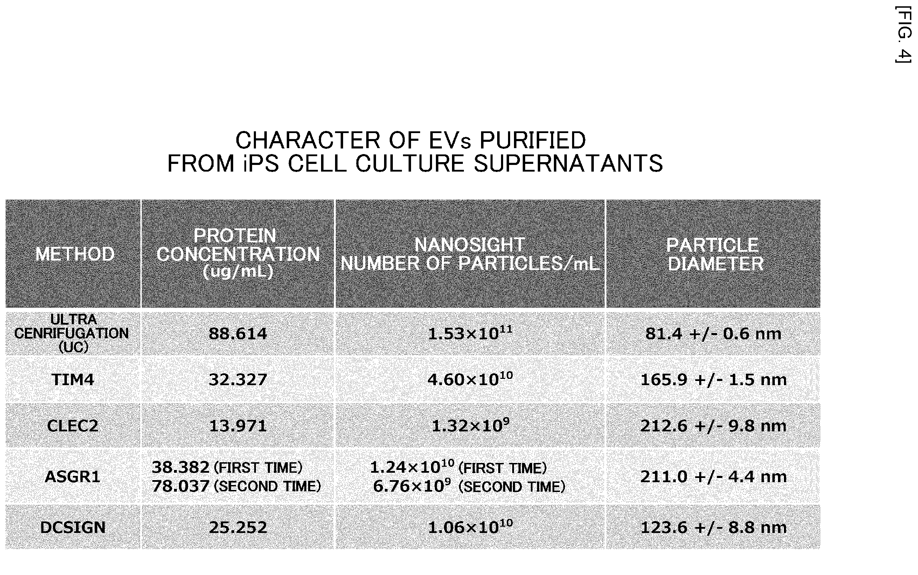

Fig. 4 shows the protein concentrations, numbers of particles per ml, and particle diameters (mean ± standard error) of extracellular vesicles separated from iPS cell culture supernatant with three C-type lectins (ASGR1, DCSIGN, and CLEC1B), an ultracentrifugation method, and a TIM4 method in Example 4.

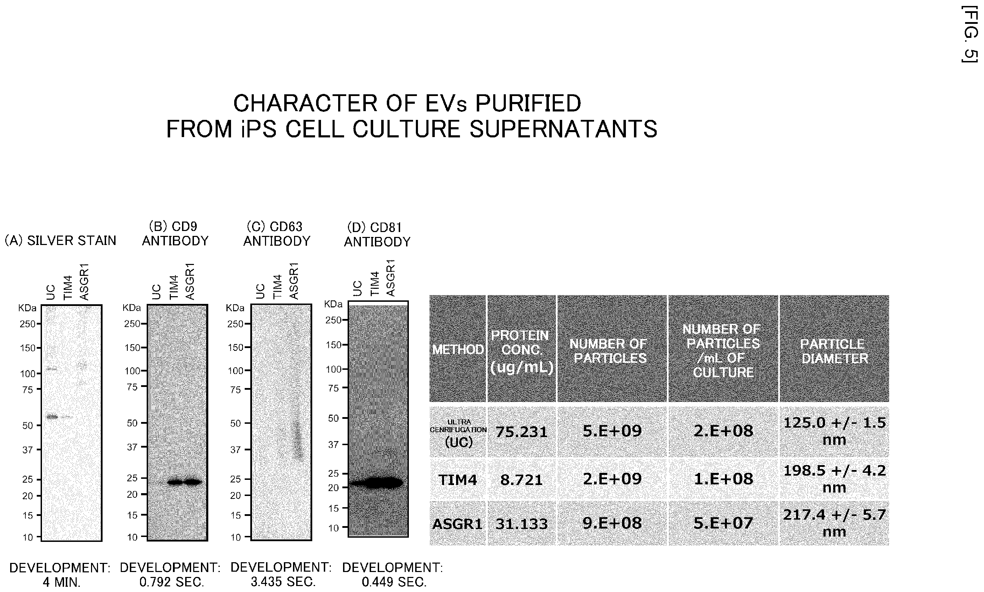

Fig. 5 shows silver staining (A) as well as western blots using an anti-CD9 antibody (B), an anti-CD63 antibody (C), and an anti-CD81 antibody (D) of extracellular vesicles separated from iPS cell culture supernatant with a C-type lectin (ASGR1), an ultracentrifugation (UC) method, and a TIM4 method in Example 5. The right figure shows the protein concentrations, numbers of particles, numbers of particles per ml, and particle diameters (mean ± standard error) of the extracellular vesicles obtained with each separation method.

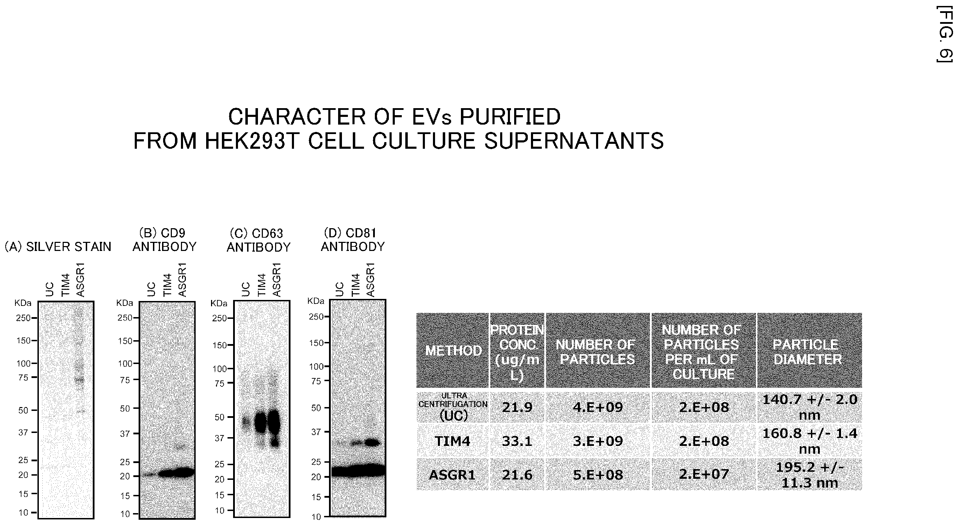

Fig. 6 shows silver staining (A) as well as western blots using an anti-CD9 antibody (B), an anti-CD63 antibody (C), and an anti-CD81 antibody (D) of extracellular vesicles separated from HEK293T cell culture supernatant with a C-type lectin (ASGR1), an ultracentrifugation (UC) method, and a TIM4 method using in Example 6. The right figure shows the protein concentrations, numbers of particles, numbers of particles per ml, and particle diameters (mean ± standard error) of the extracellular vesicles obtained with each separation method.

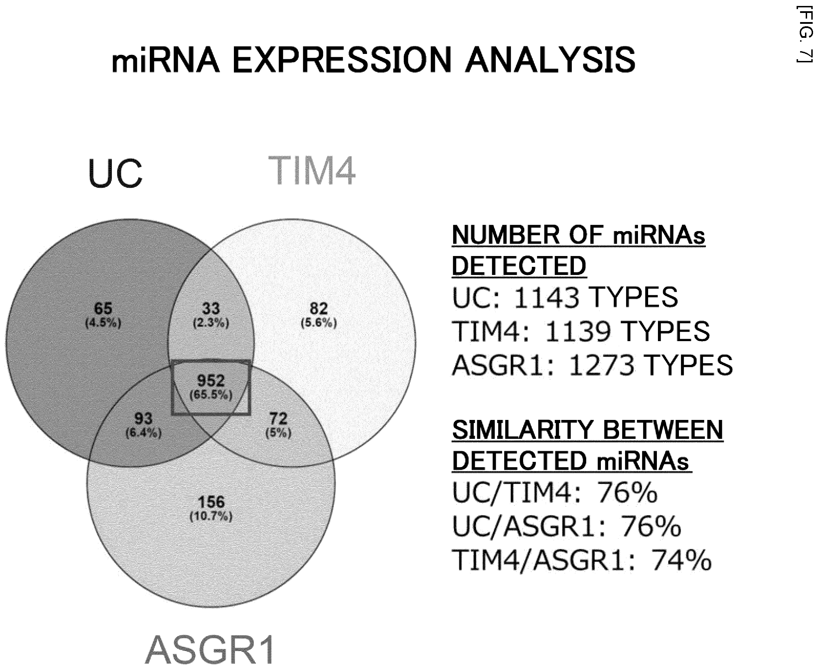

Fig. 7 shows the results of miRNA expression analysis of extracellular vesicles separated from HEK293T cell culture supernatant with a C-type lectin (ASGR1), an ultracentrifugation (UC) method, and a TIM4 method in Example 7.

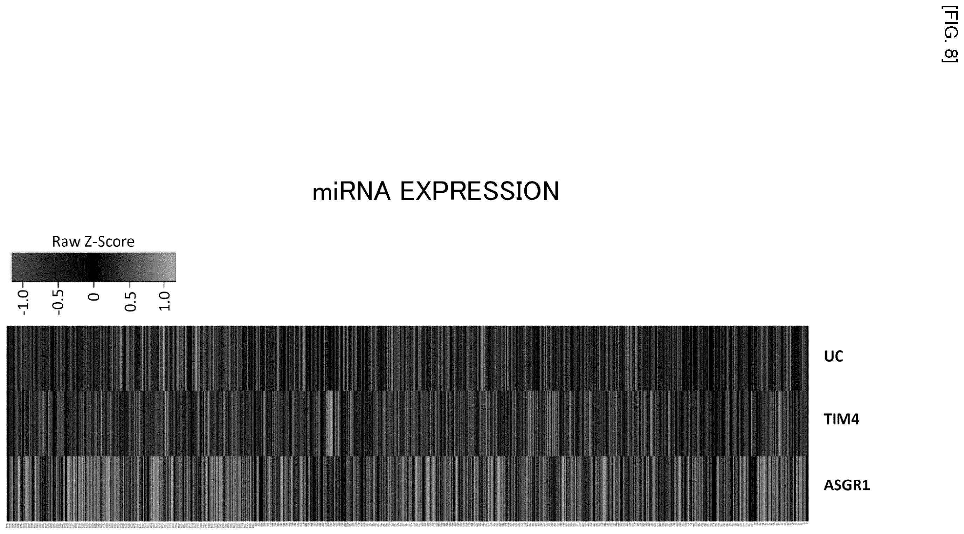

Fig. 8 shows the results of heatmap analysis of the miRNA expression amounts in extracellular vesicles separated from HEK293T cell culture supernatant with a C-type lectin (ASGR1), an ultracentrifugation (UC) method, and a TIM4 method in Example 8.

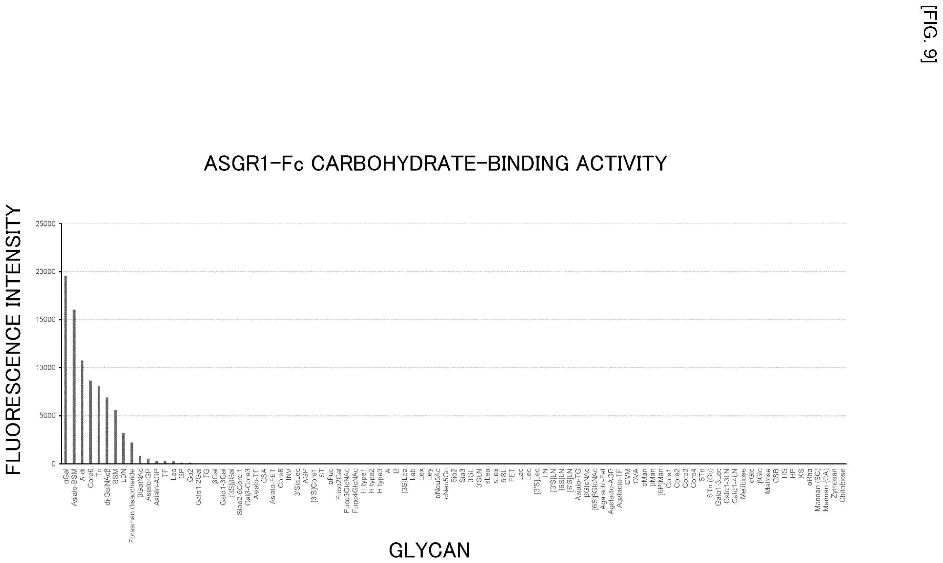

Fig. 9 shows the results of glycan microarray analysis of ASGR1 in Example 9.

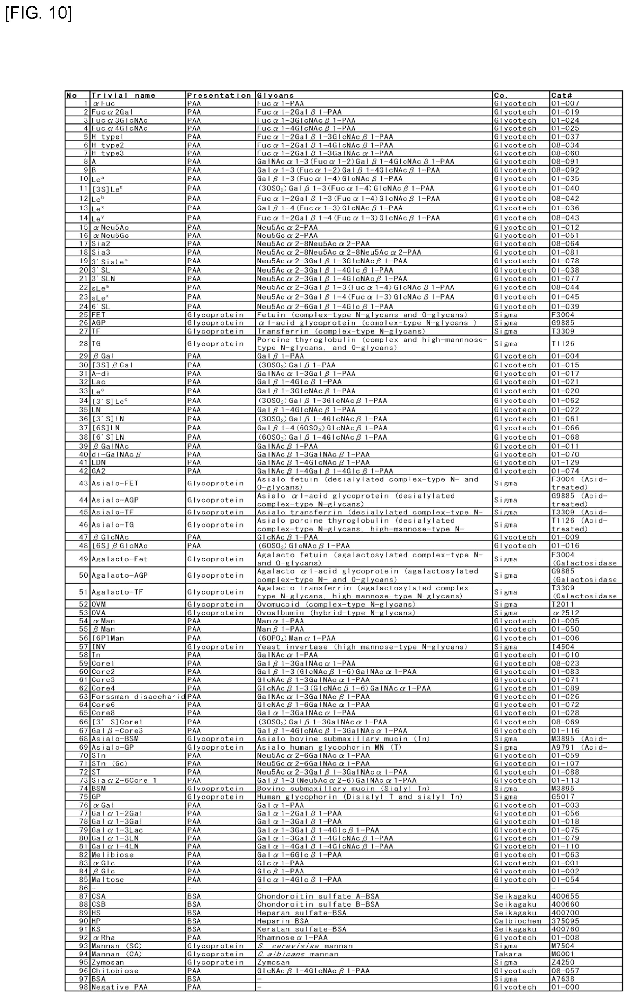

Fig. 10 shows a list of glycans used in the glycan array in Example 9.

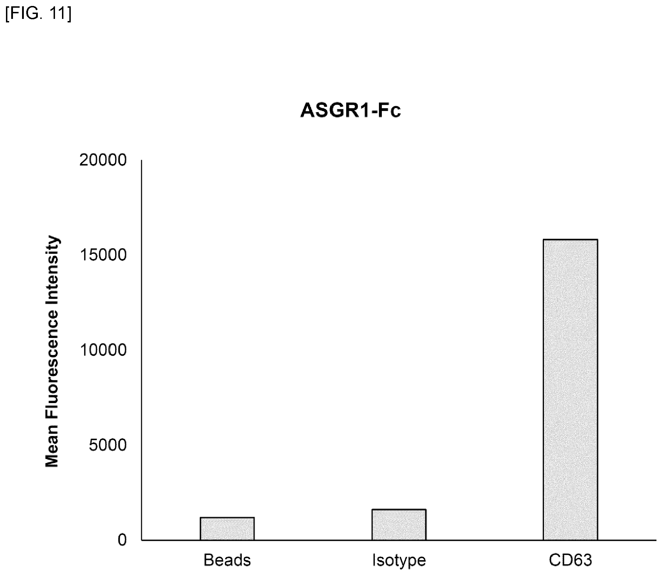

Fig. 11 is a graph showing the results of flow cytometry analysis of extracellular vesicles using ASGR1-immobilized beads. The vertical axis shows the average fluorescence intensity. "Beads" is a group in which beads to which ASGR1 is not immobilized were reacted with cell culture supernatant and reacted with an anti-CD63 antibody, "Isotype" is a group in which the ASGR1-immobilized beads were reacted with cell culture supernatant and reacted with a control antibody, and "CD63" is a group in which the ASGR1-immobilized beads were reacted with cell culture supernatant and reacted with the anti-CD63 antibody.

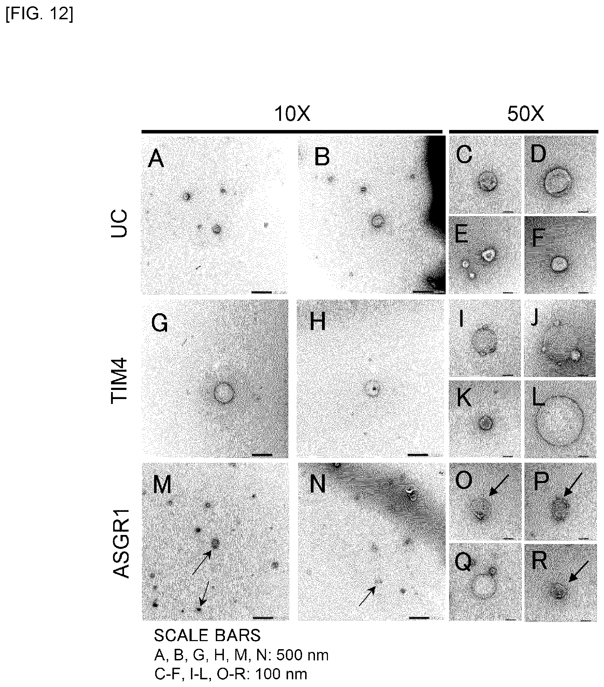

Fig. 12 is a transmission electron microscope photograph of extracellular vesicles separated from HEK293T cell culture supernatant with a C-type lectin (ASGR1) (M to R), an ultracentrifugation (UC) method (A to F), and a TIM4 method (G to L) in Example 11. The arrows indicate vesicles having a lipid bilayer. Scale bars: 500 nm (A, B, G, H, M, and N) and 100 nm (C to F, I to L, and O to R).

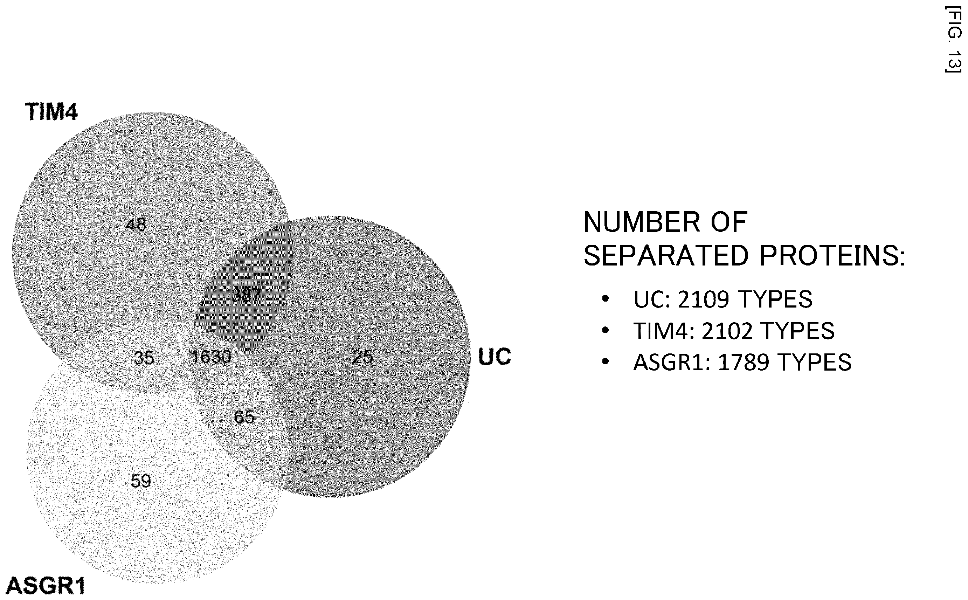

Fig. 13 shows the results of proteomic analysis of extracellular vesicles separated from HEK293T cell culture supernatant with a C-type lectin (ASGR1), an ultracentrifugation (UC) method, and a TIM4 method (analysis of the types of proteins in the extracellular vesicles separated with each separation method) in Example 12.

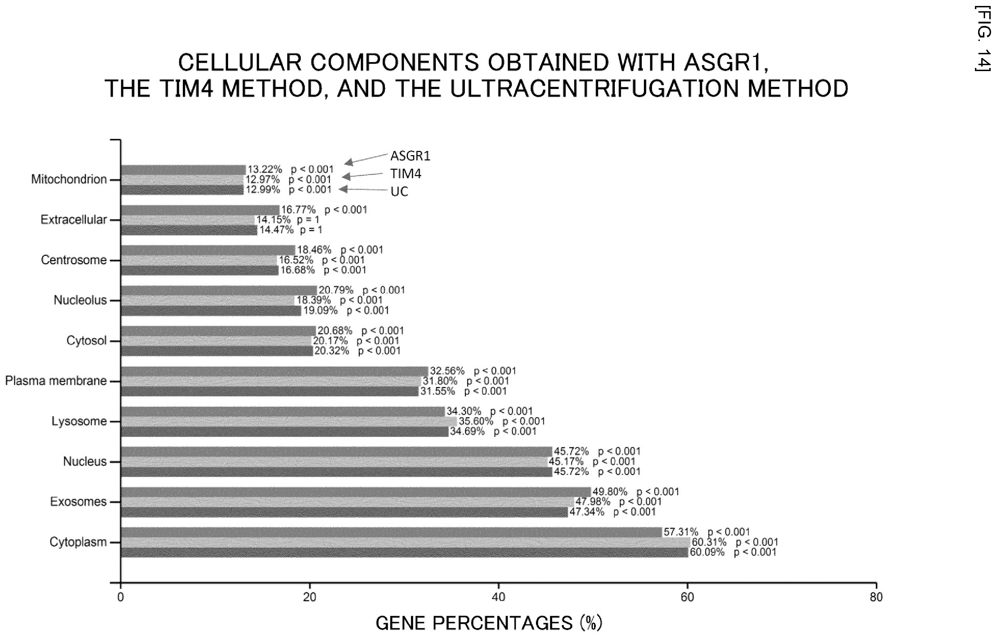

Fig. 14 shows the results of proteomic analysis of extracellular vesicles separated from HEK293T cell culture supernatant with a C-type lectin (ASGR1), an ultracentrifugation (UC) method, and a TIM4 method (analysis of the proportions (%) of proteins associated with each cellular component in the extracellular vesicles separated with each separation method) in Example 12.

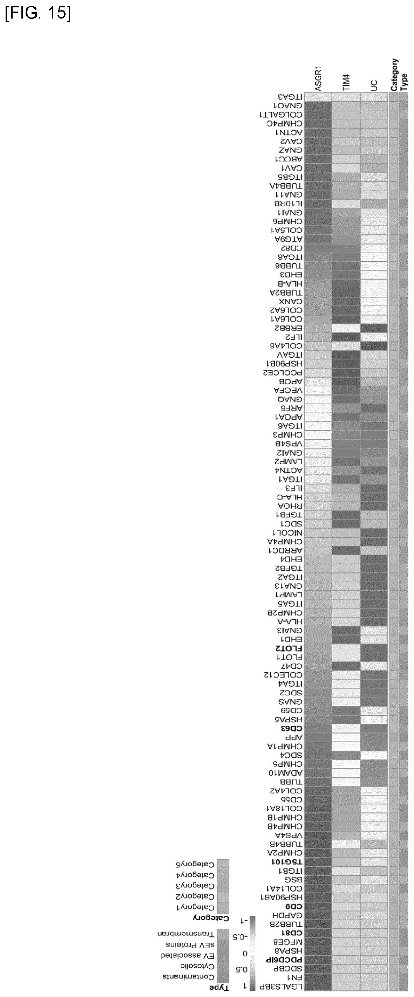

Fig. 15 shows the results of proteomic analysis of extracellular vesicles separated from HEK293T cell culture supernatant with a C-type lectin (ASGR1), an ultracentrifugation (UC) method, and a TIM4 method (heatmap analysis of the amounts of exosome markers expressed in the extracellular vesicles separated with each separation method) in Example 12.

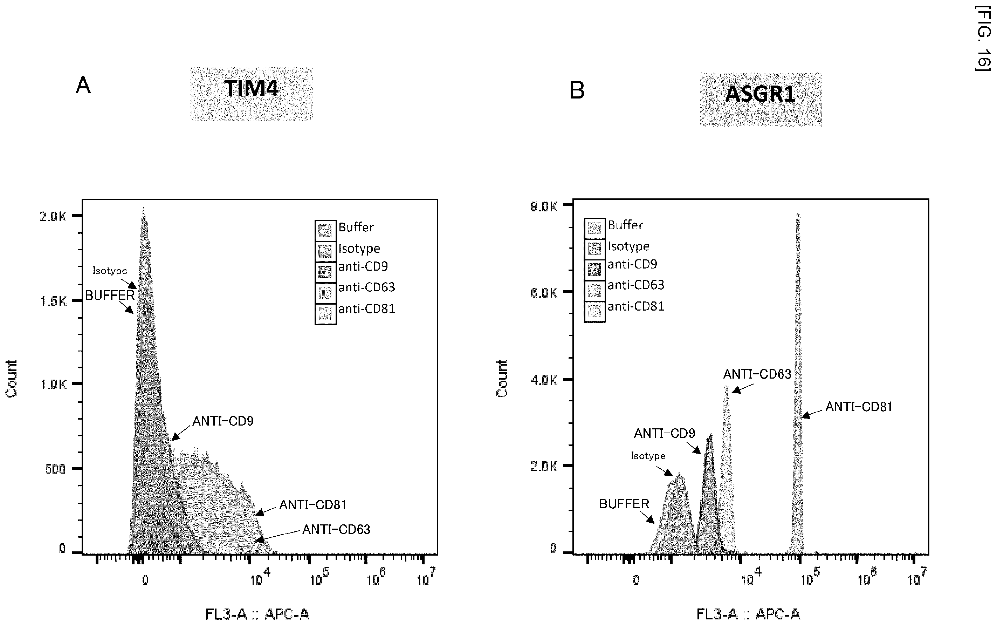

Fig. 16 is a graph showing the results of flow cytometry analysis of extracellular vesicles separated from HEK293T cell culture supernatant with TIM4-immobilized beads (A) or ASGR1-immobilized beads (B) using a buffer, a control antibody (Isotype), and exosome marker (CD9, CD63, or CD81) antibodies in Example 13.

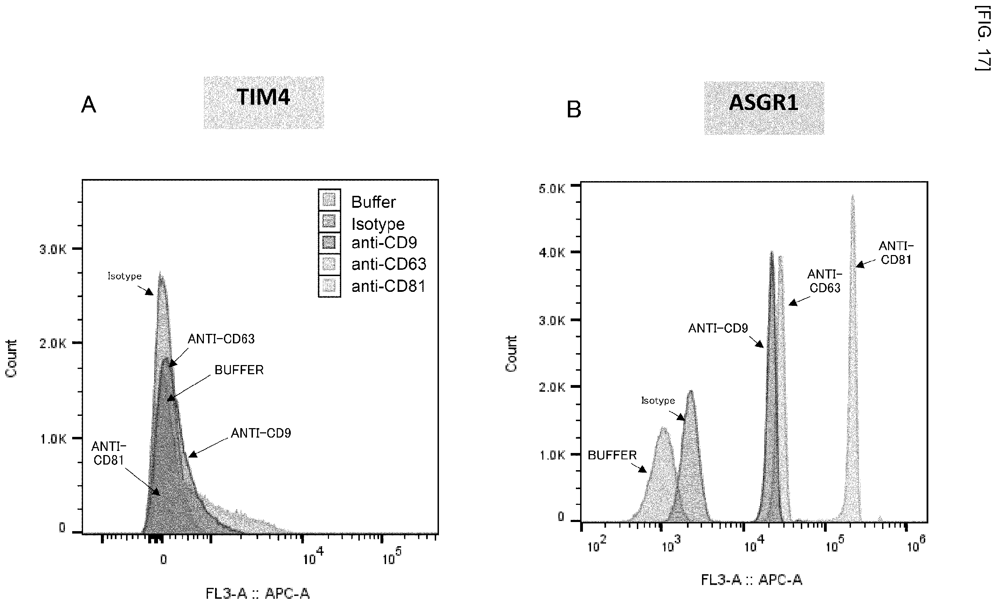

Fig. 17 is a graph showing the results of flow cytometry analysis of extracellular vesicles separated from 201B7 iPS cell culture supernatant with TIM4-immobilized beads (A) or ASGR1-immobilized beads (B) using a buffer, a control antibody (Isotype), and exosome marker (CD9, CD63, or CD81) antibodies in Example 14.

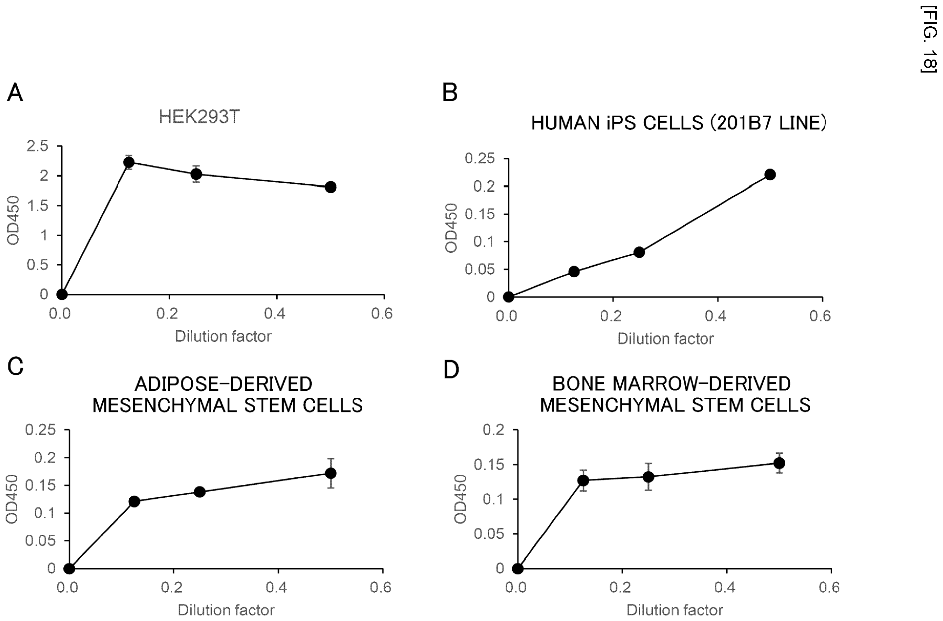

Fig. 18 is a graph showing the results of ELISA analysis in which the culture supernatant of HEK293T cells (A), 201B7 iPS cells (B), adipose-derived mesenchymal stem cells (C), or bone marrow-derived mesenchymal stem cells (D) was detected with an anti-CD63 antibody following reaction on an ASGR1-Fc-immobilized plate in Example 15.

One embodiment of the present disclosure shall be explained in detail below, but the scope of the present disclosure is not limited to the one embodiment explained herein and various modifications can be made within a scope not deviating from the gist of the present disclosure. Each of the aspects disclosed in the present description can be combined with any other feature disclosed in the present description. Further, when multiple upper and lower limits are described for a particular parameter, any upper and lower limit among these upper and lower limits can be combined to set a suitable numerical range. Moreover, the lower limits and/or upper limits of numerical ranges described in the present disclosure are numerical values within the numerical range and may be substituted with numerical values shown in the examples. The expression "X-Y" indicating a numerical range means "X or greater and Y or less". When a specific explanation that describes an embodiment also applies to another embodiment, there are cases where the explanation is omitted for the other embodiment.

When there are no particular explanations in the embodiments or examples, methods described in standard collections of protocols such as J. Sambrook, E. F. Fritsch & T. Maniatis (Ed.), Molecular cloning, a laboratory manual (3rd edition), Cold Spring Harbor Press, Cold Spring Harbor, New York (2001) and F.M. Ausubel, R. Brent, R.E. Kingston, D.D. Moore, J.G. Seidman, J.A. Smith, K. Struhl (Ed.), Current Protocols in Molecular Biology, John Wiley & Sons Ltd. or methods modifying or altering the same are used. Further, when commercially available reagent kits or measuring devices are used, the protocol included therewith is used in cases where there is no particular explanation.

### == First Embodiment (Separation Method) ==

The separation method according to the present embodiment is a method for separating extracellular vesicles from a sample including the extracellular vesicles that uses a C-type lectin.

In one embodiment, the separation method is for separating the extracellular vesicles in the sample from other components in the sample.

### (Extracellular Vesicles)

Extracellular vesicles in the present description encompass vesicles which are variously classified according to their origin, the size of the small membrane vesicles, etc.

Specifically, extracellular vesicles in the present description encompass exosomes, microvesicles, apoptotic vesicles, adiposomes, and the like.

Exosomes are small membrane vesicles that are derived from late endosomes, are composed of a lipid bilayer, and have phosphatidylserine on their membrane surfaces. The diameter of the vesicles is usually from about 50 nm to about 250 nm. Exosomes are known to include tetraspanins such as CD9, CD63, and CD81 and proteins such as Alix, TSG101, Lamp-1, and Flotillin (Thery et al., "Minimal information for studies of extracellular vesicles 2018 (MISEV2018): a position statement of the International Society for Extracellular Vesicles and update of the MISEV2014 guidelines", Journal of Extracellular Vesicles, 2018, Vol. 7, 1535750, https://doi.org/10.1080/20013078.2018.1535750). It is said that there are subclasses of exosomes that further differ in production mechanism and function.

Microvesicles are small membrane vesicles that are derived from plasma membranes, are composed of a lipid bilayer, and have phosphatidylserine on their membrane surfaces. Microvesicles are normally about 100 nm to about 1000 nm in size. Microvesicles are known to include proteins such as integrins, selectins, and CD40 ligand.

Apoptotic vesicles are small membrane vesicles that are derived from apoptotic cells, are composed of a lipid bilayer, and have phosphatidylserine on their membrane surfaces. Apoptotic vesicles are normally about 50 nm to about 500 nm in size. Apoptotic vesicles are known to include histones.

Adiposomes are small membrane vesicles that are derived from adipose cells, are composed of a lipid bilayer, and have phosphatidylserine on their membrane surfaces. Adiposomes are normally about 100 nm to about 1000 nm in size. Adiposomes are known to include MFG-E8 (milk fat globule-EGF factor 8).

Whether extracellular vesicles are naturally occurring or artificially synthesized, the extracellular vesicles are subject to separation with the separation method according to the present embodiment. Further, the extracellular vesicles included in the sample may include extracellular vesicles other than the subject to be separated, and the extracellular vesicles other than the subject to be separated may also be naturally occurring extracellular vesicles or artificially synthesized extracellular vesicles.

In cases where the extracellular vesicles are naturally occurring, the cells from which the extracellular vesicles are derived are not limited to the scope of those that secrete extracellular vesicles, may be plant cells, animal cells, or microbial cells, and are not limited by the species of origin, the developmental origin, the tissue or organ of origin, cellular function, or the like. The plant cell may be, for example, a cell derived from either a seed plant or a non-seed plant, or may be a cell derived from any organ, such as a stem, leaf, flower, or root. The microbial cell may be, for example, either a prokaryotic or a eukaryotic cell, and more specific non-limiting examples include E. coli, staphylococcus, halophiles, acidophiles, thermophiles, yeasts, fungi, bacteria, and the like. The animal cell may be a cell derived from, for example, any of a mammal, a bird, a reptile, an amphibian, a fish, or the like, may be endoderm-derived, ectoderm-derived, mesoderm-derived, or a stem cell, and may be epithelial tissue-derived, connective tissue-derived, muscle tissue-derived, or nerve tissue-derived. Non-limiting examples of cells include blood cells, muscle cells, skin cells, nerve cells, glandular cells, and the like. These cells may be primary cells taken from an individual organism or tissue, or may be cultured cells.

More specific non-limiting examples of cells from which the extracellular vesicles are derived include, for instance, various stem cells such as mesenchymal stem cells (MSCs) (which encompass adipose-derived mesenchymal stem cells and bone marrow-derived mesenchymal stem cells), induced pluripotent stem cells (iPS cells), and embryonic stem cells (ES cells), cell lines (HEK293 cells, CHO cells, HeLa cells, MCF-7 cells, COS cells, PC12 cells, etc.), and primary cultured cells (skin fibroblasts, endothelial cells, myocytes, hematopoietic cells, etc.).

In one embodiment, the cell from which the extracellular vesicles are derived may be a human-derived stem cell, a cell line established from a human cell, or a human-derived primary cultured cell.

Naturally occurring extracellular vesicles also encompass extracellular vesicles in which, as compared with extracellular vesicles found in nature, the amount of glycans with low specificity to C-type lectins is reduced or these are absent, or in which the proportion of glycans with high specificity to C-type lectins is increased by performing treatment on extracellular vesicles separated from plant cells, animal cells, microbial cells, etc. with an enzyme that acts on the glycans or the like. Non-limiting examples of enzymatic treatment are sialidases and galactosidases.

Artificially synthesized extracellular vesicles can be obtained by culturing a plant-cultured cell, an animal-cultured cell, or a microorganism-cultured cell that secretes extracellular vesicles in an appropriate culture medium under appropriate conditions and causing the extracellular vesicles to be secreted into the culture medium. The artificially synthesized extracellular vesicles may be extracellular vesicles where, for example, knockout treatment of certain genes associated with glycan synthesis or the like is carried out in a plant-cultured cell, an animal-cultured cell, or a microorganism-cultured cell and the amount of glycans with low specificity to C-type lectins of the secreted extracellular vesicles is reduced or these are absent, or in which the proportion of glycans with high specificity to C-type lectins is increased as compared with cases in which genetic engineering has not been carried out. Non-limiting examples of genetic engineering are knockout of core 1 synthase (C1GALT1) and knockout of Cosmc, which is a molecular chaperone of C1GALT1.

In one embodiment, at least a portion of the extracellular vesicles included in the sample may be extracellular vesicles that are the subject to be separated and in this case, the extracellular vesicles that are the subject to be separated have glycans with binding to C-type lectins and the other extracellular vesicles do not have glycans with binding to C-type lectins. Therefore, the extracellular vesicles having binding to C-type lectins can be separated with the separation method according to the present embodiment, which includes a step for contacting the extracellular vesicles in the sample with a C-type lectin and separating the extracellular vesicles bound to the C-type lectin from the C-type lectin.

In one embodiment, the extracellular vesicles that are the subject to be separated are at least CD9-positive, CD63-positive, and/or CD81-positive extracellular vesicles. In one embodiment, the extracellular vesicles that are the subject to be separated are at least CD9-positive, CD63-positive, and CD81-positive extracellular vesicles.

The at least CD9-positive, CD63-positive, and/or CD81-positive extracellular vesicles express the glycans described in "C-type Lectin" below on their surfaces and are therefore readily separated from the sample including the extracellular vesicles with the separation method using a C-type lectin according to the present embodiment.

### (Sample)

In the present embodiment, the extracellular vesicles are separated from the sample comprising the extracellular vesicles using a C-type lectin. The sample is not limited within the scope of including extracellular vesicles and may be a biological sample or experimental sample taken from a living body.

The biological sample is, for example, a body fluid sample, a solid sample, or a semisolid sample in which the extracellular vesicles are present. Here, bodily fluids also include bodily fluids in a broad sense, for example, blood (plasma, serum, and whole blood), digestive fluids such as gastric juice, bile, pancreatic fluid, and intestinal fluid, tissue fluids such as interstitial fluid, intercellular fluid, and stromal interstitial fluid, coelomic fluid, serosal fluid, pleural fluid, ascites, pericardial fluid, cerebrospinal fluid, joint fluid (synovial fluid), aqueous humor, lymphatic fluid, amniotic fluid, saliva, sputum, urine, semen, sweat, tears, nasal mucus, vaginal fluid, milk, etc. Further, solid samples include, for example, tissue slices taken from an individual organism, feces, etc. Semisolid samples include vomit, etc. Other examples of biological samples include plant or fruit extracts, cell culture media, microbial culture media, and the like. Culture solutions encompass those which include cells or microbes and supernatants thereof. The microbial culture medium may be, for example, a yeast culture medium and more specifically may be an alcohol product including mash in an alcohol manufacturing process or at least a portion of components of the mash, or including extracellular vesicles derived from the mash.

The species from which the biological sample is derived is not limited to the scope of those having extracellular vesicles, and may be an animal, a plant, or a microbial cell. The species is the same as that of the cell from which the extracellular vesicles are derived, as described in the section "Extracellular Vesicles".

When the sample is a biological sample, the sample may be stored after being taken from the subject until implementation of the separation method in accordance with methods well known to those skilled in the art.

Non-limiting examples of experimental samples include culture supernatants including extracellular vesicles, physiological saline solutions including extracellular vesicles, buffers including extracellular vesicles, and the like. The extracellular vesicles may be artificially synthesized extracellular vesicles as explained in the section "Extracellular Vesicles" above.

### (C-type Lectins)

Lectin is typically a generic term for substances that have specific binding activity mainly for glycans among proteins or glycoproteins present in plants, animals, microorganisms, etc. C-type lectins are one of the major structurally defined lectin families and broadly encompass lectins that bind to glycans and ligands other than glycans, regardless of the presence or absence of calcium dependence.

Considering the binding to the extracellular vesicles that are the subject to be separated, the C-type lectin in the present embodiment is preferably a lectin having calcium-dependent glycan binding activity and is more preferably a C-type lectin receptor (transmembrane receptor). The C-type lectin receptor is not limited by the structure thereof and may be, for example, any C-type lectin receptor classified as Group 2, Group 4, Group 5, Group 6, Group 14, etc. The C-type lectin receptor is not limited by other functions within the scope of having binding affinity for extracellular vesicles, nor is it limited by the level of binding to extracellular vesicles.

Binding between the C-type lectin receptor and extracellular vesicles means the binding between the C-type lectin receptor and glycans expressed on the extracellular vesicle surfaces. The glycan is not limited within the scope of being expressed on the extracellular vesicle surface in a form capable of binding to C-type lectins, nor is it limited by its expression level.

In one embodiment, the glycan is that with αGalNAc or βGalNAc at the non-reducing end. Non-limiting examples of glycans having αGalNAc or βGalNAc at the non-reducing end include type A glycan (GalNAcα1-3Galβ1-4GlcNAcβ1-), Core 6 (GlcNAcβ1-6GalNAcα1-), di-GalNAcβ (GalNAcβ1-3GalNAcβ1-), LDN (GalNAcβ1-4GlcNAcβ1-), Forssman (GalNAcα1-3GalNAcβ1-3Galα1-4Galβ1-4Glcβ1-), Forssman disaccharide (GalNAcα1-3GalNAcβ1-), βGalNAc (GalNAcβ1-), Tn (GalNAcα1-), A-di (GalNAcα1-3Galβ1-), GA2 (GalNAcβ1-4Galβ1-4Glcβ1-), GM2 (GalNAcβ1-4(Siaa2-3)Galβ1-4Glcβ1-), GD2 (GalNAcβ1-4(Siaa2-8Siaα2-3)Galβ1-4Glcβ1-Cer), and the like.

In one embodiment, the glycan is αGal (Galα1-).

In one embodiment, the glycan may be present as a glycoprotein, and examples of such glycoproteins include bovine submaxillary mucin (BSM), asialo-BSM, and the like.

In another embodiment, the glycan may be LeX (Galβ1-4(Fucα1-3)GlcNAc), LeA (Galβ1-3(Fucα1-4)GlcNAc), a high mannose-type glycan, type O glycan (di-sialyl T(Siaα2-3Galβ1-3(Siaα2-6)GalNAc), etc.), or the like.

That is, considering the binding between the C-type lectin and the extracellular vesicles, the C-type lectin receptor is preferably a C-type lectin receptor having binding to one or more glycans of type A glycan (GalNAcα1-3Galβ1-4GlcNAcβ1-), Core 6 (GlcNAcβ1-6GalNAca1-), Tn (GalNAcα1-), di-GalNAcβ (GalNAcβ1-3GalNAcβ1-), LDN (GalNAcβ1-4GlcNAcβ1-), Forssman (GalNAcα1-3GalNAcβ1-3Galα1-4Galβ1-4Glcβ1-), βGalNAc (GalNAcβ1-), Tn (GalNAcα1-), A-di (GalNAcα1-3Galβ1-), BSM (sialylated Tn), αGal (Galα1-), BSM (bovine submaxillary mucin), asialo-BSM, LeX (Galβ1-4(Fucα1-3)GlcNAc), LeA (Galβ1-3(Fucα1-4)GlcNAc), high mannose-type glycans, type O glycan (di-sialyl T(Siaα2-3Galβ1-3(Siaα2-6)GalNAc), etc., and may be a C-type lectin receptor having binding to one or more glycans of type A glycan (GalNAcα1-3Galβ1-4GlcNAcβ1-), Core 6 (GIcNAcβ1-6GalNAcα1-), Tn (GalNAcα1-), di-GalNAcβ (GalNAcβ1-3GalNAcβ1-), LDN (GalNAcβ1-4GlcNAcβ1-), Forssman (GalNAcα1-3GalNAcβ1-3Galα1-4Galβ1-4Glcβ1-), βGalNAc (GalNAcβ1-), Tn (GalNAcα1-), A-di (GalNAcα1-3Galβ1-) and/or one or more glycoproteins of BSM (sialylated Tn), αGal (Galα1-), BSM (bovine submaxillary mucin), and asialo-BSM.

The presence or absence of binding between the C-type lectin receptor and these glycans can be assessed with methods well known to those skilled in the art, for example with a glycan array.

Considering the binding with the extracellular vesicles that are the subject to be separated, the C-type lectin receptor is more preferably ASGR1 (asialoglycoprotein receptor 1 (UniProt No. P07306)), DCSIGN (CD209 antigen (UniProt No. Q9NNNX6)), or CLEC1B (C-type lectin domain family 1 member B (UniProt No. Q9P126)), and still more preferably is ASGR1. Due to the C-type lectin receptor being ASGR1, DCSIGN, and/or CLEC1B, CD9-positive, CD63-positive, and/or CD81-positive extracellular vesicles are readily separated with the separation method according to the present embodiment. Due to the C-type lectin receptor being ASGR1, CD9-positive, CD63-positive, and/or CD81-positive extracellular vesicles are readily separated at a higher purity.

From the perspective of the types of separable extracellular vesicle, there is the advantage of extracellular vesicles of different types than the extracellular vesicles obtainable with conventional methods (for example,TIM4 methods, ultracentrifugation methods, etc.) being more readily separated due to the use of the C-type lectin receptor. Specifically, extracellular vesicles that encapsulate and/or express genes (for example, miRNA) and/or proteins different from those in conventional methods are readily separated due to the use of the C-type lectin receptor. From the perspective of the types of separable extracellular vesicles, the C-type lectin receptor is preferably ASGR1, DCSIGN and/or CLEC1B, and is more preferably ASGR1.

It being possible to separate different types of extracellular vesicles enables analysis and use thereof for disease diagnosis and/or treatment.

The C-type lectin is not limited by the preparation method therefor and may be a naturally occurring lectin or an artificially synthesized lectin. In the case of a naturally occurring lectin, the lectin may be isolated from biological tissues or cells of plants, animals, microorganisms, etc. according to methods well known to those skilled in the art. In the case of an artificially synthesized lectin, the lectin can be prepared with techniques well known to those skilled in the art, such as expressing a recombinant lectin in mammalian cells, plant cells, yeast, or E. coli. The lectin can also be prepared with an *in vitro* translation system. The C-type lectin may be a commercially available lectin provided by reagent companies such as ACROBiosystems, R&D Systems, Inc., Sigma-Aldrich, Takara Bio Inc., and FUJIFILM Wako Pure Chemical Corporation.

The C-type lectin encompasses proteins or peptides other than full-length proteins within the scope of maintaining binding to the glycan to be bound and may be, for example, a structure in which a portion of a protein has been deleted, a structure in which an arbitrary peptide has been added to a full-length protein, an extracellular domain, a protein or peptide including an extracellular domain, a carbohydrate-binding domain, a protein or peptide including a carbohydrate-binding domain, or the like.

The C-type lectin may be immobilized on any carrier to suit the separation method according to the present embodiment. The carrier can be selected as appropriate from carriers well known to those skilled in the art and may be in the form of a microplate, a tube, a disk-like piece, a particle (bead), or the like. When the carrier is a particle or the like, a column for purification or separation may be filled with the carrier. The carrier may be a porous body having through-holes such as a sponge monolith. The size of the through-holes is not limited and may be about 1 nm to about 100 µm. Due to the carrier being a material having a large surface area such as a porous body, separation efficiency in the separation method readily improves. The material of the carrier can also be selected as appropriate from substances well known to those skilled in the art, and may be polystyrene, polyacrylic acid, polymethacrylic acid, polymethyl methacrylate, polyacrylamide, polyglycidyl methacrylate, polypropylene, polyolefins, polyimides, polyurethanes, polyesters, polyvinyl chloride, polyethylene, polychlorocarbonates, silicone resins, silicone rubbers, agarose, dextran, glass, silicon oxide, diatom, porous glass, ground glass, alumina, silica gel, metal oxides, iron, cobalt, nickel, magnetite, chromite, or the like.

For example, in the case of a particle (bead), the size of the particle can be selected as appropriate by those skilled in the art in accordance with the application and may be, for example, about 10 nm to about 100 µm, about 100 nm to about 10 µm, or about 1 µm to about 5 µm.

Methods of immobilizing the C-type lectin on the carrier can be selected as appropriate from methods well known to those skilled in the art and the C-type lectin may be, for example, bound by affinity binding, bound by chemical binding, or bound by physical adsorption. More specifically for example, the Fc region of an antibody may be added to a lectin and, using the binding force between the Fc region and protein G, the lectin bound to beads to which protein G has been added, a biotinylated lectin may be immobilized on streptavidin-immobilized beads, or a lectin may be immobilized on beads that have been activated by n-hydroxysuccinimide (NHS) or an epoxy group with amine coupling.

### (Separation Method)

In one embodiment, the separation method is a separation method for separating extracellular vesicles in a sample, including a step of contacting the extracellular vesicles in the sample with a C-type lectin and a step of separating the extracellular vesicles bound to the C-type lectin from the C-type lectin.

The sample including the extracellular vesicles is as explained in "Sample" above and may be a biological sample or experimental sample taken from a living body. Either sample may be pre-treated prior to the separation method according to the present embodiment. The pre-treatment may be, for example, enrichment of the extracellular vesicles by removing undesired substances in the sample or the like. More specifically, the substances in the sample can be selected according to the size or properties thereof and the undesired substances removed by ultrafiltration, tangential flow filtration, ultracentrifugation, ion exchange separation, or the like.

Contact between the extracellular vesicles in the sample and the C-type lectin can be made by mixing the C-type lectin and the sample including the extracellular vesicles or the like. For example, when the C-type lectin is immobilized on a carrier such as a microplate, tube, or disk-like piece, the C-type lectin may be mixed by dripping a liquid sample including the extracellular vesicles onto the side of the carrier on which the C-type lectin is exposed. A C-type lectin immobilized on a particulate carrier such as beads and a sample including extracellular vesicles may be mixed in a single solution. Alternatively, a non-immobilized C-type lectin and a sample including extracellular vesicles may be mixed in a single solution.

At least a portion of the mixed C-type lectin and at least a portion of the extracellular vesicles will thereby bind due to the binding thereof and form a complex.

The solution used in the step of contacting the extracellular vesicles in the sample with the C-type lectin is not limited within the scope wherein the extracellular vesicles and the C-type lectin can be dissolved or suspended in a stable state and contact and/or binding between the C-type lectin and the extracellular vesicles is not hindered, and examples thereof include, for instance, water or a buffer with a pH of about 7.0 to about 8.0, preferably about 7.2 to about 7.6 (for example, Tris buffer, HEPES buffer, etc.).

In one embodiment, the step of contacting the extracellular vesicles in the sample with the C-type lectin is preferably performed in the presence of calcium ions. The C-type lectin according to the present embodiment is preferably a lectin having calcium-dependent glycan-binding activity because the binding to the extracellular vesicles improves due to the presence of calcium ions in this case. When calcium ions are present, using a buffer that does not bind calcium and result in precipitate is preferred.

The origin of the calcium ions is not limited and the calcium ions may be derived from, for example, calcium chloride, calcium hydroxide, calcium hydrogen carbonate, calcium iodide, calcium bromide, calcium acetate, or the like. The calcium ions are preferably derived from calcium chloride, calcium hydrogen carbonate, or calcium iodide and more preferably derived from calcium chloride or calcium hydrogen carbonate. The calcium ion concentration can be adjusted by adding one or more of these ion compounds prior to or at the same time the extracellular vesicles in the sample and the C-type lectin are contacted, and the final concentration thereof is about 0.5 to about 100 mM, is preferably about 0.5 to about 10 mM, is more preferably about 0.5 to about 5.0 mM, and is still more preferably about 0.5 to about 2.0 mM. When the final concentration is about 0.5 to about 2.0 mM, the final concentration may be about 1.0 mM.

The step of contacting the extracellular vesicles in the sample and the C-type lectin can be performed by incubating at about 10 °C or less, preferably at about 1 to about 6 °C, and more preferably at about 4 °C for about 0.5 to about 24 hours, preferably about 3 to about 20 hours, and more preferably about 6-12 hours.

In one embodiment, when a non-immobilized C-type lectin and a sample including extracellular vesicles have been mixed in a single solution, a carrier such as beads that specifically bind to the C-type lectin may be introduced to the complex in which the C-type lectin and the extracellular vesicles are bound to form a complex of the C-type lectin, the carrier, and the extracellular vesicles.

Separation of the extracellular vesicles bound to the C-type lectin from the C-type lectin can be carried out by incubating the complex of the C-type lectin and the extracellular vesicles in an elution buffer.

At least a portion of the extracellular vesicles bound to the C-type lectin is thereby separated from the C-type lectin.

The solution used in this step is not limited within the scope wherein the extracellular vesicles and the C-type lectin can be dissolved or suspended in a stable state and separation of the C-type lectin and the extracellular vesicles is not hindered, and examples thereof include, for instance, water or a buffer with a pH of about 7.0 to about 8.0, preferably about 7.2 to about 7.6 (for example, Tris buffer, HEPES buffer, etc.).

In one embodiment, the elution buffer is preferably free of calcium ions. The C-type lectin according to the present embodiment is preferably a lectin having calcium-dependent glycan-binding activity, because the extracellular vesicles are more readily separated from the C-type lectin due to the absence of calcium ions.

In one embodiment, the elution buffer may be that including a glycan that the C-type lectin recognizes. This is because the efficiency of separation of the extracellular vesicles from the C-type lectin readily improves due to the use of such an elution buffer. The extracellular vesicles are still more readily separated from the C-type lectin by using an elution buffer which is free of calcium ions and includes a glycan that the C-type lectin recognizes.

Such a glycan may be a glycan that the C-type lectin recognizes according to the C-type lectin used and, for example, when the C-type lectin is ASGR1, DCSIGN, or CLEC1B, the glycan is preferably that having αGalNAc, βGalNAc, αMan, αFuc, or di-sialyl T (Siaα2-3Galβ1-3(Siaα2-6)GalNAc) at the non-reducing end. Examples of αGalNAc and βGalNAc are as described in "C-type Lectin" above.

In one embodiment, the separation method according to the present embodiment may include a step of washing the complex and removing undesired substances prior to the step of separating the extracellular vesicles bound to the C-type lectin from the C-type lectin in the elution buffer. The washing is preferably performed about 1 to about 3 times and may be performed twice. The washing buffer is not limited within the scope wherein the extracellular vesicles and C-type lectin can be dissolved or suspended in a stable state and separation of the C-type lectin and the extracellular vesicles is not hindered, and examples thereof include, for instance, water or a buffer having a pH of about 7.0 to about 8.0, preferably about 7.2 to about 7.6 (for example, Tris buffer, HEPES buffer, etc.). For example, when the C-type lectin is immobilized on a carrier such as a microplate, tube, or disk-like piece, washing can be performed by dripping the washing buffer onto a surface on the carrier where the complex of the C-type lectin and the extracellular vesicles is present. For example, when the C-type lectin is immobilized on beads, washing can be performed by adding the washing buffer to the complex of the bead-immobilized C-type lectin and the extracellular vesicles and then, having used the beads to aggregate, precipitate, etc., removing the supernatant. For example, when the beads are magnetic beads, the complex including the beads can be aggregated or precipitated by magnetic force.

The separation method according to the present embodiment can separate extracellular vesicles having binding to a C-type lectin.

In one embodiment, when ASGR1, DCSIGN, or CLEC1B is used as the C-type lectin, extracellular vesicle populations including at least CD9-positive, CD63-positive, and/or CD81-positive extracellular vesicles can be separated.

In one embodiment, when ASGR1, DCSIGN, or CLEC1B is used as the C-type lectin, extracellular vesicle populations including at least CD9-positive, CD63-positive, and CD81-positive extracellular vesicles can be separated.

In one embodiment, the average particle diameter of the extracellular vesicles separated with the separation method of the present embodiment may be about 50 nm to about 250 nm, may be about 100 nm to about 250 nm, may be about 150 nm to about 250 nm, or may be about 200 nm to about 250 nm.

In cases where, for example, the C-type lectin is immobilized on a carrier, the extracellular vesicles separated from the C-type lectin that are the subject to be separated can be isolated from the sample by acquiring only the solution including the extracellular vesicles in a free state.

The separation method according to the present embodiment can be used in a method for purifying extracellular vesicles by further isolating extracellular vesicles which have been separated from other components in a sample with the separation method, as described above.

Further, the separation method can also be used in a method for detecting extracellular vesicles by labeling the separated extracellular vesicles with a label that binds to a marker specific for the extracellular vesicles (for example, a specific antibody).

In one embodiment, the population of extracellular vesicles separated with the separation method includes at least CD9-positive, CD63-positive, and/or CD81-positive extracellular vesicles at a high density as compared with the sample including the extracellular vesicles prior to subjection to the separation method.

In one embodiment, the population of extracellular vesicles separated with the separation method includes at least CD9-positive, CD63-positive, and CD81-positive extracellular vesicles at a high density as compared with the sample including the extracellular vesicles prior to subjection to the separation method.

In one embodiment, at least CD9-positive, CD63-positive, and/or CD81-positive extracellular vesicles or at least CD9-positive, CD63-positive, and CD81-positive extracellular vesicles can be concentrated with the separation method according to the present embodiment.

Furthermore, the separation method according to the present embodiment can, by using extracellular vesicles purified with the same, be used in the diagnosis of symptoms, diseases, etc. in a subject based on analysis of bioinformation molecules included in the extracellular vesicles. That is, in one embodiment, a diagnostic method includes the separation method according to the present embodiment and preferably includes a step of analyzing information molecules in the separated extracellular vesicles.

Examples of symptoms or diseases in a subject include cancer, neurodegenerative diseases, psychiatric disorders, chronic diseases (lifestyle-related diseases), and the like, but are not limited thereto.

### == Second Embodiment (Kit) ==

The kit according to the present embodiment is a kit for separating extracellular vesicles from a sample including the extracellular vesicles, wherein the kit includes a C-type lectin.

In one embodiment, the kit is for separating the extracellular vesicles in the sample from other components in the sample.

In the present embodiment, examples and preferred examples of the "extracellular vesicles", the "C-type lectin", and the "sample" are as described in the first embodiment.

The kit according to the present embodiment can be used to perform any of the separation methods described in the first embodiment.

In one embodiment, in addition to the C-type lectin, the kit may include one or more reagents necessary for steps in the separation methods described in the first embodiment, that is, a "step of contacting the extracellular vesicles in the sample with the C-type lectin" and/or a "step of separating the extracellular vesicles bound to the C-type lectin from the C-type lectin".

The reagent is, for example, a buffer, an ion compound including calcium ions, a glycan used in the elution buffer, and/or a washing buffer used in these steps. In the kit, the C-type lectin may be immobilized on a carrier as described in the first embodiment, but in cases where the C-type lectin is not immobilized, the kit may include a carrier for immobilizing the C-type lectin and one or more reagents for immobilizing the C-type lectin on the carrier.

In one embodiment, the kit can be used as a kit for purifying extracellular vesicles by further isolating extracellular vesicles which have been separated from other components in a sample with the separation method. Further, the kit can also be used as a kit for detecting extracellular vesicles by labeling the separated extracellular vesicles with a label that binds to a marker specific for the extracellular vesicles (for example, a specific antibody). In this case, the kit may include a means for labeling the marker specific for the extracellular vesicles. Such a means may be an antibody which specifically binds to a marker expressed specifically by the extracellular vesicles. The antibody is preferably an anti-CD9 antibody, an anti-CD63 antibody, and/or an anti-CD81 antibody. In one embodiment, the kit includes a C-type lectin and an antibody that specifically binds to the extracellular vesicles.

The antibody which specifically binds to a marker expressed specifically by the extracellular vesicles may be that to which modifications necessary for detection thereof have been added, and the modifications can be selected, as appropriate, from features well known to those skilled in the art and may be, for example, biotin, a fluorescent material, an enzyme, or the like.

The kit for detecting extracellular vesicles may be, for example, for EIAs (Enzyme Immunoassays) including chemiluminescence enzyme immunoassays (CLEIAs), fluorescence immunoassays, ELISAs (Enzyme-Linked Immunosorbent Assays), RIAs (Radioimmunoassays), western blot methods, latex agglutination methods, immunochromatography methods, sandwich methods, flow cytometry methods, etc.

The kit according to the present embodiment and various kits using the same include a C-type lectin as described above and further include: one or more reagents as described above, a means for labeling with markers specific for the extracellular vesicles, positive/negative controls, and/or instructions for use of the kit.

The kit for separating extracellular vesicles in a sample according to the present embodiment and various kits using the same can be used as kits for using extracellular vesicles purified with the kit and diagnosing symptoms, diseases, etc. in a subject based on analysis of bioinformation molecules included in the extracellular vesicles.

Further, a kit for detecting extracellular vesicles can be used for the quantification of extracellular vesicles in a sample and the symptoms or disease in a subject can be diagnosed based on the results thereof. That is, the kit for detecting extracellular vesicles according to the present embodiment can be used as a kit for diagnosing symptoms or a disease in a subject based on the detection or quantification results thereof.

Examples of symptoms or diseases in a subject include cancer, neurodegenerative diseases, psychiatric disorders, chronic diseases (lifestyle-related diseases), and the like, but are not limited thereto.

### EXAMPLES

The present invention shall be explained more specifically with reference to examples below, but the interpretation of the present disclosure is not limited by these examples.

Exosome CD9 Antibody (Ts9) (Invitrogen, 10626D), Anti-CD63 Human (Mouse) Unlabeled 8A12 (Cosmo Bio Co., Ltd., SHI-EXO-MO2), and Anti-CD81 Human (Mouse) Unlabeled 12C4 (Cosmo Bio Co., Ltd, SHI-EXO-MO3) were used as antibodies against CD9, CD63, and CD81 throughout the following examples.

Further, unless specifically noted, the analyses were performed according to the methods described in the previous examples.

### [Example 1] Separation of Extracellular Vesicles with a TIM4 Method

1 µg of galactose-binding lectin rLSLN (a recombinant N-terminal domain of Laetiporus sulphureus-derived lectin) was mixed with 10 µl of Dynabeads M280 Streptavidin (VERITAS Corporation) and bound for 30 minutes at 4 °C to produce rLSLN lectin-immobilized beads.

50 ml of the culture supernatant of 201B7 iPS cells was concentrated to 1 ml with 100 kDa cut Amicon Ultra (Merck Ltd.). This was then mixed with the rLSLN-immobilized beads and reacted overnight at 4 °C. After washing four times with 1 ml of PBS, the mixture was eluted with a 0.2 M lactose solution.

For use as a positive control, the extracellular vesicles were separated using the MagCapture^{™} Exosome Isolation Kit PS (TIM4 method, FUJIFILM Wako Pure Chemical Corporation) according to the instructions.

Protein quantification was performed on the obtained extracellular vesicles using a micro-BCA assay (Pierce Biotechnology Inc.), and the particle diameter and number of particles were measured with NanoSight (Quantum Design Japan). Furthermore, after electrophoresis of 7.4×10⁷ particles of each sample, the samples were transferred to PVDF membranes and western blotting was performed with antibodies against CD9, CD63, and CD81.

The western blot results are shown in Fig. 1.

The extracellular vesicles separated with the TIM4 method were positive for CD9, CD63, and CD81. Accordingly, extracellular vesicle populations including at least CD9-positive, CD63-positive, and/or CD81-positive extracellular vesicles were able to be separated. Meanwhile, the extracellular vesicles separated with the rLSLN-immobilized beads were negative for CD9, CD63, and CD81 and it was not possible to separate CD9-positive, CD63-positive, or CD81-positive extracellular vesicles.

### [Example 2] Separation of Extracellular Vesicles using a Human C-type Lectin

The base sequences encoding the extracellular domains of human C-type lectins (Table 1, SEQ ID NOs: 1-23) were ligated to the Xhol/EcoRV restriction enzyme site in pSecTag/FRT/V5-His TOPO vector (Thermo Fisher Scientific Inc.). Furthermore, expression vectors were produced by ligating human IgG1 Fc regions to the Agel/Pmel restriction enzyme site. HEK293T cells were transfected with the produced expression vectors with Lipofectamine LTX Reagent with PLUS Reagent (Thermo Fisher Scientific Inc.), cultured in a CO₂ incubator, and the culture supernatant was collected. Human C-type lectin-Fc fusion proteins were purified from the collected culture supernatant using rProtein A Sepharose Fast Flow (GE HealthCare Japan). Alongside confirming the degree of purification with electrophoresis and western blotting of the purified proteins, the amount of proteins was quantified with a Pierce^{™} BCA Protein Assay Kit (Thermo Fisher Scientific Inc.). A total of 23 human C-type lectin-Fc fusion proteins was produced. TIM4 and the other 23 kinds of human C-type lectins used in the examples are as shown in Figure 2.

**Table 1**

| **ABBREVIATION** | **UNIPROT NO.** | **MANUFACTURER** | **SEQ ID NO.** | **AMINO ACID SEQUENCE** |
|---|---|---|---|---|
| P-SELECTIN | P16109 | ACRO BIOSYSTEMS (CAT NO. SEP-H5255) | 1 | WTYHYSTKAYSWNISRKYCQNRYTDLVAIQNKNEIDYLNKVLPYYSSYYWIGIRK NNKTWTWVGTKKALTNEAENWADNEPNNKRNNEDCVEIYIKSPSAPGKWNDE HCLKKKHALCYTASCQDMSCSKQGECLETIGNYTCSCYPGFYGPECEYVRECGEL ELPQHVLMNCSHPLGNFSFNSQCSFHCTDGYQVNGPSKLECLASGIWTNKPPQC LAAQCPPLKIPERGNMTCLHSAKAFQHQSSCSFSCEEGFALVGPEVVQCTASGV WTAPAPVCKAVQCQHLEAPSEGTMDCVHPLTAFAYGSSCKFECQPGYRVRGLD MLRCIDSGHWSAPLPTCEAISCEPLESPVHGSMDCSPSLRAFQYDTNCSFRCAEG FMLRGADIVRCDNLGQWTAPAPVCQALQCQDLPVPNEARVNCSHPFGAFRYQS VCSFTCNEGLLLVGASVLQCLATGNWNSVPPECQAIPCTPLLSPQNGTMTCVQPL GSSSYKSTCQFICDEGYSLSGPERLDCTRSGRWTDSPPMCEAIKCPELFAPEQGS LDCSDTRGEFNVGSTCHFSCNNGFKLEGPNNVECTTSGRWSATPPTCKGIASLPT PGVQCPALTTPGQGTMYCRHHPGTFGFNTTCYFGCNAGFTLIGDSTLSCRPSGQ WTAVTPACRAVKCSELHVNKPIAMNCSNLWGNFSYGSICSFHCLEGQLLNGSAQ TACQENGHWSTTVPTCQAGPLTIQEA |
| ASGR1 | P07306 | NATIONAL INSTITUTE OF ADVANCED INDUSTRIAL SCIENCE AND TECHNOLOGY (AIST) | 2 | LSCQMAALQGNGSERTCCPVNWVEHERSCYWFSRSGKAWADADNYCRLEDAH LVVVTSWEEQKFVQHHIGPVNTWMGLHDQNGPWKWVDGTDYETGFKNWRPE QPDDWYGHGLGGGEDCAHFTDDGRWNDDVCQRPYRWVCETELDKASQEPPLL |
| DCSIGN | Q9NNX6 | AIST | 3 | SKVPSSISQEQSRQDAIYQNLTQLKAAVGELSEKSKLQEIYQELTQLKAAVGELPE KSKLQEIYQELTRLKAAVGELPEKSKLQEIYQELTWLKAAVGELPEKSKMQEIYQE LTRLKAAVGELPEKSKQQEIYQELTRLKAAVGELPEKSKQQEIYQELTRLKAAVGE LPEKSKQQEIYQELTQLKAAVERLCHPCPWEWTFFQGNCYFMSNSQRNWHDSIT ACKEVGAQLVVIKSAEEQNFLQLQSSRSNRFTWMGLSDLNQEGTWQWVDGSPL LPSFKQYWNRGEPNNVGEEDCAEFSGNGWNDDKCNLAKFWICKKSAASCSRDE EQFLSPAPATPNPPPA |
| LANGERIN | Q9UJ71 | AIST | 4 | DILQVVSQGWKYFKGNFYYFSLIPKTWYSAEQFCVSRNSHLTSVTSESEQEFLYK TAGGLIYWIGLTKAGMEGDWSWVDDTPFNKVQSVRFWIPGEPNNAGNNEHCG NIKAPSLQAWNDAPCDKTFLFICKRPYVPSEP |
| ASGR2 | P07307 | AIST | 5 | VACQMELLHSNGSQRTCCPVNWVEHQGSCYWFSHSGKAWAEAEKYCQLENAH LVVINSWEEQKFIVQHTNPFNTWIGLTDSDGSWKWVDGTDYRHNYKNWAVTQ PDNWHGHELGGSEDCVEVQPDGRWNDDFCLQVYRWVCEKRRNATGEVA |
| PROLECTIN | Q6ZS10 | AIST | 6 | LDCRRITCPEGWLPFEGKCYYFSPSTKSWDEARMFCQENYSHLVIINSFAEHNFV AKAHGSPRVYWLGLNDRAQEGDWRWLDGSPVTLSFWEPEEPNNIHDEDCATM NKGGTWNDLSCYKTTYWICERKCSC |
| LSECTIN | Q6UXB4 | AIST | 7 | ASTERAALLDGHDLLRTNASKQTAALGALKEEVGDCHSCCSGTQAQLQTTRAEL GEAQAKLMEQESALRELRERVTQGLAEAGRGREDVRTELFRALEAVRLQNNSCE PCPTSWLSFEGSCYFFSVPKTTWAAAQDHCADASAHLVIVGGLDEQGFLTRNTR GRGYWLGLRAVRHLGKVQGYQWVDGVSLSFSHWNQGEPNDAWGRENCVMML HTGLWNDAPCDSEKDGWICEKRHNC |
| DCSIGNR | Q9H2X3 | AIST | 8 | SKVPSSLSQEQSEQDAIYQNLTQLKAAVGELSEKSKLQEIYQELTQLKAAVGELPE KSKLQEIYQELTRLKAAVGELPEKSKLQEIYQELTRLKAAVGELPEKSKLQEIYQEL TRLKAAVGELPEKSKLQEIYQELTELKAAVGELPEKSKLQEIYQELTQLKAAVGELP DQSKQQQIYQELTDLKTAFERLCRHCPKDWTFFQGNCYFMSNSQRNWHDSVTA CQEVRAQLVVIKTAEEQNFLQLQTSRSNRFSWMGLSDLNQEGTWQWVDGSPLS PSFQRYWNSGEPNNSGNEDCAEFSGSGWNDNRCDVDNYWICKKPAACFRDE |
| MINCLE | Q9ULY5 | AIST | 9 | RCVVTFRIFQTCDEKKFQLPENFTELSCYNYGSGSVKNCCPLNWEYFQSSCYFFS TDTISWALSLKNCSAMGAHLVVINSQEEQEFLSYKKPKMREFFIGLSDQVVEGQ WQWVDGTPLTKSLSFWDVGEPNNIATLEDCATMRDSSNPRQNWNDVTCFLNY FRICEMVGINPLNKGKSL |
| DCIR | Q9UMR7 | AIST | 10 | QKYSQLLEKKTTKELVHTTLECVKKNMPVEETAWSCCPKNWKSFSSNCYFISTES ASWQDSEKDCARMEAHLLVINTQEEQDFIFQNLQEESAYFVGLSDPEGQRHWQ WVDQTPYNESSTFWHPREPSDPNERCVVLNFRKSPKRWGWNDVNCLGPQRSV CEMMKIHL |
| DECTIN1 | Q9BXN2 | AIST | 11 | TMAIWRSNSGSNTLENGYFLSRNKENHSQPTQSSLEDSVTPTKAVKTTGVLSSP CPPNWIIYEKSCYLFSMSLNSWDGSKRQCWQLGSNLLKIDSSNELGFIVKQVSSQ PDNSFWIGLSRPQTEVPWLWEDGSTFSSNLFQIRTTATQENPSPNCVWIHVSVI YDQLCSVPSYSICEKKFSM |
| DECTIN2 | Q6EIG7 | AIST | 12 | TYHFTYGETGKRLSELHSYHSSLTCFSEGTKVPAWGCCPASWKSFGSSCYFISSE EKVWSKSEQNCVEMGAHLVVFNTEAEQNFIVQQLNESFSYFLGLSDPQGNNNW QWIDKTPYEKNVRFWHLGEPNHSAEQCASIVFWKPTGWGWNDVICETRRNSIC EMNKIYL |
| DECTIN3 | Q8WXI8 | AIST | 13 | CLVTHHNFSRCKRGTGVHKLEHHAKLKCIKEKSELKSAEGSTWNCCPIDWRAFQ SNCYFPLTDNKTWAESERNCSGMGAHLMTISTEAEQNFIIQFLDRRLSYFLGLRD ENAKGQWRWVDQTPFNPRRVFWHKNEPDNSQGENCVVLVYNQDKWAWNDV PCNFEASRICKIPGTTLN |
| MDL1 | Q9NY25 | AIST | 14 | PQIFNKSNDGFTTTRSYGTVSQIFGSSSPSPNGFITTRSYGTVCPKDWEFYQARC FFLSTSESSWNESRDFCKGKGSTLAIVNTPEKLKFLQDITDAEKYFIGLIYHREEKR WRWINNSVFNGNVTNQNQNFNCATIGLTKTFDAASCDISYRRICEKNAK |
| CD301 | Q8IUN9 | AIST | 15 | QNSKFQRDLVTLRTDFSNFTSNTVAEIQALTSQGSSLEETIASLKAEVEGFKQERQ AGVSELQEHTTQKAHLGHCPHCPSVCVPVHSEMLLRVQQLVQDLKKLTCQVATL NNNASTEGTCCPVNWVEHQDSCYWFSHSGMSWAEAEKYCQLKNAHLVVINSR EEQNFVQKYLGSAYTWMGLSDPEGAWKWVDGTDYATGFQNWKPGQPDDWQG HGLGGGEDCAHFHPDGRWNDDVCQRPYHWVCEAGLGQTSQESH |
| CLEC12A (DCAL2) | Q5QGZ9 | AIST | 16 | |
| CLEC9A (CD370) | Q6UXN8 | AIST | 17 | KLLQVSTIAMQQQEKLIQQERALLNFTEWKRSCALQMKYCQAFMQNSLSSAHNS SPCPNNWIQNRESCYYVSEIWSIWHTSQENCLKEGSTLLQIESKEEMDFITGSLR KIKGSYDYWVGLSQDGHSGRWLWQDGSSPSPGLLPAERSQSANQVCGYVKSNS LLSSNCSTWKYFICEKYALRSSV |
| CLEC8A (LOX1) | P78380 | AIST | 18 | |
| CLEC1B (CLEC2) | Q9P126 | AIST | 19 | SVMQRNYLQGENENRTGTLQQLAKRFCQYVVKQSELKGTFKGHKCSPCDTNWR YYGDSCYGFFRHNLTWEESKQYCTDMNATLLKIDNRNIVEYIKARTHLIRWVGLS RQKSNEVWKWEDGSVISENMFEFLEDGKGNMNCAYFHNGKMHPTFCENKHYL MCERKAGMTKVDQLP |
| MRC1 | P22897 | AIST | 20 | TRQFLIYNEDHKRCVDAVSPSAVQTAACNQDAESQKFRWVSESQIMSVAFKLCL GVPSKTDWVAITLYACDSKSEFQKWECKNDTLLGIKGEDLFFNYGNRQEKNIML YKGSGLWSRWKIY |
| CLEC2D | Q9UHP7 | AIST | 21 | RANCHQEPSVCLQAACPESWIGFQRKCFYFSDDTKNWTSSQRFCDSQDADLAQ VESFQELNFLLRYKGPSDHWIGLSREQGQPWKWINGTEWTRQFPILGAGECAYL NDKGASSARHYTERKWICSKSDIHV |
| L-SELECTIN | P14151 | AIST | 22 | WTYHYSEKPMNWQRARRFCRDNYTDLVAIQNKAEIEYLEKTLPFSRSYYWIGIRK IGGIWTWVGTNKSLTEEAENWGDGEPNNKKNKEDCVEIYIKRNKDAGKWNDDA CHKLKAALCYTASCQPWSCSGHGECVEIINNYTCNCDVGYYGPQCQFVIQCEPLE APELGTMDCTHPLGNFSFSSQCAFSCSEGTNLTGIEETTCGPFGNWSSPEPTCQV IQCEPLSAPDLGIMNCSHPLASFSFTSACTFICSEGTELIGKKKTICESSGIWSNPS PICQKLDKSFSMIKEGDYN |
| E-SELECTIN | P16581 | ACRO BIOSYSTEMS (CAT NO. CDE-H5250) | 23 | WSYNTSTEAMTYDEASAYCQQRYTHLVAIQNKEEIEYLNSILSYSPSYYWIGIRK VNNVWVWVGTQKPLTEEAKNWAPGEPNNRQKDEDCVEIYIKREKDVGMWNDE RCSKKKLALCYTAACTNTSCSGHGECVETINNYTCKCDPGFSGLKCEQIVNCTAL ESPEHGSLVCSHPLGNFSYNSSCSISCDRGYLPSSMETMQCMSSGEWSAPIPACN VVECDAVTNPANGFVECFQNPGSFPWNTTCTFDCEEGFELMGAQSLQCTSSGN WDNEKPTCKAVTCRAVRQPQNGSVRCSHSPAGEFTFKSSCNFTCEEGFMLQGPA QVECTTQGQWTQQIPVCEAFQCTALSNPERGYMNCLPSASGSFRYGSSCEFSCE QGFVLKGSKRLQCGPTGEWDNEKPTCEAVRCDAVHQPPKGLVRCAHSPIGEFTY KSSCAFSCEEGFELHGSTQLECTSQGQWTEEVPSCQVVKCSSLAVPGKINMSCS GEPVFGTVCKFACPEGWTLNGSAARTCGATGHWSGLLPTCEAPTESNIP |

C-type lectin-immobilized beads were produced by binding 12 µg of a C-type lectin-Fc fusion protein to 50 µl of Dynabeads Protein G (VERITAS Corporation) for 30 minutes at 4 °C. 50 ml of the culture supernatant of 201B7 iPS cells cultured with mTeSR PLUS (VERITAS Corporation) was collected and concentrated to 1 ml with 100 kDa cut Amicon Ultra (Merck Ltd.). CaCl₂ was added such that the final concentration became 1 mM, and the supernatant was mixed with the C-type lectin-immobilized beads and incubated overnight at 4 °C while being mixed. After spin-down with a benchtop centrifuge, the mixture was reacted with a magnetic stand to remove the culture supernatant. 1 ml of washing buffer (10 mM Tris, 137 mM NaCl, pH 7.4, 1 mM CaCl₂, and 0.02% Tween20) was then added and after vortexing, reacted with a magnetic stand to remove the supernatant. After repeating the washing operation twice and the supernatant was removed, incubation with 50 µl of elution buffer (10 mM Tris and 137 mM NaCl, pH 7.4 with 1mM EDTA) at room temperature for 10 min. and elution were performed. The elution operation was repeated twice to prepare a solution including 100 µl of extracellular vesicles.

For use as a positive control, the extracellular vesicles were separated using the MagCapture^{™} Exosome Isolation Kit PS (TIM4 method, FUJIFILM Wako Pure Chemical Corporation) according to the instructions.

Protein quantification was performed on the obtained extracellular vesicles using a micro-BCA assay (Pierce Biotechnology Inc.), and the particle diameter and number of particles were measured with NanoSight (Quantum Design Japan). Furthermore, after electrophoresis of 36 µl of each sample, the samples were transferred to PVDF membranes and western blotting was performed with antibodies against CD9, CD63, and CD81.

### The results are shown in Fig. 2.

As shown in "Stats: Mean+/- Standard Error" in Figure 2, 200 nm or smaller extracellular vesicles that demonstrate reactivity to antibodies against CD9, CD63, and CD81 were able to be separated with the TIM4 method. Further, about 100 nm to about 250 nm extracellular vesicles were able to be separated with many C-type lectin-immobilized beads. Among these, populations of extracellular vesicles including those demonstrating reactivity to antibodies against the tetraspanins CD9, CD63, and CD81 were able to be separated with ASGR1, DCSIGN, and CLEC1B.

The western blot bands were evaluated as follows:
· No reactivity to an antibody detected: 1
· Reactivity to an antibody detected: 2
· Strong reactivity to an antibody detected: 3

As shown in Figure 2, reactivity to the anti-CD9 antibody and the anti-CD63 antibody and strong reactivity to the anti-CD81 antibody were found in the extracellular vesicles separated with TIM4.

Reactivity to the anti-CD9 antibody and the anti-CD63 antibody and strong reactivity to the anti-CD81 antibody were found in the extracellular vesicles separated with DCSIGN.

Reactivity to the anti-CD9 antibody and the anti-CD63 antibody and strong reactivity to the anti-CD81 antibody were found in the extracellular vesicles separated with ASGR1.

Reactivity to the anti-CD9 antibody and the anti-CD63 antibody and strong reactivity to the anti-CD81 antibody were found in the extracellular vesicles separated with CLEC1B.

No reactivity to the anti-CD9 antibody, the anti-CD63 antibody, or the anti-CD81 antibody was detected with lectins other than the above.

### [Example 3] Marker Expression in Extracellular Vesicles Separated using a Human C-type Lectin

The extracellular vesicles prepared in Example 2 were electrophoresed at 0.15 µg/well each and western blotting with antibodies against CD9, CD63, and CD81 was performed. The results thereof are, as shown in Figure 3, that the populations of extracellular vesicles separated with three kinds of C-type lectin (ASGR1, DCSIGN, and CLEC1B)-immobilized beads included CD9-positive, CD63-positive, and/or CD81-positive extracellular vesicles.

### [Example 4] Comparison of Extracellular Vesicles Separated with Different Separation Methods

Furthermore, the separation of extracellular vesicles using three C-type lectin (ASGR1, DCSIGN, and CLEC1B)-immobilized beads was investigated and compared with extracellular vesicles separated with ultracentrifugation and TIM4 methods.

C-type lectin-immobilized beads were prepared with the same method as in Example 2, and extracellular vesicles were separated with the C-type lectin-immobilized beads and a TIM4 method. In the ultracentrifugation (UC) method, 20 ml of iPS cell culture supernatant was filtered with a 0.22 µm filter and then placed in an ultracentrifugation tube, and ultracentrifugation was performed in an ultracentrifuge (Beckman Optima MAX-XP) for 70 minutes at 100,000× g and 4 °C. After removal of the supernatant, a small amount of PBS was added to the ultracentrifugation tube and agitated with vortexing to collect elute including the extracellular vesicles.

As shown in Fig. 4, extracellular vesicles with a particle diameter (mean value) of about 100 nm to about 250 nm were able to be separated using three kinds of C-type lectin (ASGR1, DCSIGN, and CLEC1B)-immobilized beads.

### [Example 5] Separation of iPS Cell-derived Extracellular Vesicles with ASGR1

Among the three kinds of C-type lectins (ASGR1, DCSIGN, and CLEC1B), ASGR1 was further investigated. With the same method as in Example 2, a solution (100 µl) including extracellular vesicles that were separated from 201B7 iPS cell culture supernatant (20 ml) with ASGR1-immobilized beads and equal numbers of particles of extracellular vesicles (4.5×10⁷ particles/well) separated with an ultracentrifugation method and a TIM4 method were electrophoresed, and western blotting with antibodies against CD9, CD63, and CD81 was performed.

The results are shown in Fig. 5.

The results are that the extracellular vesicles separated with ASGR1-immobilized beads had a particle diameter of about 220 nm and the highest reactivity to antibodies against CD9, CD63, and CD81 as compared with the extracellular vesicles separated with the TIM4 method or ultracentrifugation method. Therefore, it was shown that for the same number of particles of extracellular vesicles, more marker (CD9, CD63, or CD81)-positive intact extracellular vesicles with a particle diameter (mean value) of about 220 nm were separated using ASGR1 as compared with the ultracentrifugation method or TIM4 method. It was thereby understood that CD9-positive, CD63-positive, and/or CD81-positive extracellular vesicles can be separated with greater purity as compared with the ultracentrifugation method or TIM4 method when ASGR1 is used.

### [Example 6] Separation of HEK293T Cell-derived Extracellular Vesicles with ASGR1

HEK293T cells were cultured with RPMI 1640, 20 ml of the obtained culture supernatant was collected, and extracellular vesicles (100 µl as solution) were separated with ASGR1-immobilized beads, a TIM4 method, and an ultracentrifugation method. The results are that extracellular vesicles with a particle diameter of about 200 nm were able to be separated with ASGR1-immobilized beads in the same manner as the ultracentrifugation method or the TIM4 method. Furthermore, equal particle numbers (4.5×10⁷ particles/well) were electrophoresed and western blotting with antibodies against CD9, CD63, and CD81 was performed.

The results are shown in Fig. 6.

The results are that the extracellular vesicles separated with ASGR1-immobilized beads had a particle diameter (mean value) of about 200 nm and the highest reactivity to antibodies against CD9, CD63, and CD81 as compared with extracellular vesicles separated with the TIM4 method or ultracentrifugation method. Therefore, it was shown that for the same number of particles of extracellular vesicles, more marker (CD9, CD63, or CD81)-positive extracellular vesicles with a particle diameter (mean value) of about 200 nm were separated using ASGR1 as compared with the ultracentrifugation method or TIM4 method. It was thereby understood that CD9-positive, CD63-positive, and/or CD81-positive extracellular vesicles can be separated with greater purity as compared with the ultracentrifugation method or TIM4 method when ASGR1 is used.

### [Example 7] miRNA Expression Analysis in Extracellular Vesicles Separated with Different Separation Methods

RNA was purified from 1×10⁹ extracellular vesicle fractions respectively separated with an ultracentrifugation (UC) method, a TIM4 method, and ASGR1, and miRNA expression analysis was performed using 3D-Gene DNA chip (TORAY INDUSTRIES, INC.).

The results are shown in Fig. 7.

1143 types of miRNA were detected from extracellular vesicles purified with the ultracentrifugation (UC) method, 1139 types were detected from extracellular vesicles purified with the TIM4 method, and 1273 types were detected from extracellular vesicles purified with ASGR1. The most miRNAs were detected from the extracellular vesicles purified with ASGR1. When the similarity of the detected miRNAs was analyzed, the similarity of the miRNAs in the extracellular vesicles purified with ASGR1 was 76% to those purified with the ultracentrifugation method and 74% to those purified with the TIM4 method. While extracellular vesicles including the same types of miRNA can be separated, it was found that there were also miRNAs which were only detected in the extracellular vesicles purified with ASGR1. These results indicate that extracellular vesicle populations different from those obtainable with ultracentrifugation methods or TIM4 methods can be obtained by using a C-type lectin (ASGR1).

### [Example 8] Heatmap Analysis of miRNA Quantities in Extracellular Vesicles Separated with Different Separation Methods

The amounts of miRNAs in extracellular vesicles purified with the three methods of an ultracentrifugation (UC) method, a TIM4 method, and ASGR1 were analyzed with Heatmapper (http://www.heatmapper.ca/) and shown with a heatmap.

The results are shown in Fig. 8.

It was understood that more miRNAs are included in the extracellular vesicles purified with ASGR1 as compared with extracellular vesicles purified with other methods.

### [Example 9] Analysis of ASGR1 Glycan Affinity with a Glycan Microarray

ASGR1 was mixed with the antibody Cy3-conjugated AffiniPure Goat Anti-Human IgG, Fcγ fragment specific (Jackson ImmunoResearch Inc.) and analyzed with a glycan microarray (Tateno et al. Glycobiology, Vol. 18, Issue 10, pp. 789-798, 2008).

As shown in Figure 9, it was understood that ASGR1 demonstrates high reactivity with αGal, Asialo-BSM (bovine submaxillary mucin), A-di (GalNAcα1-3Galβ1-PAA), Core 6 (GlcNAcB1-6GalNAcα1-), Tn (GalNAcα1-), di-GalNAcβ (GalNAcβ1-3GalNAcβ1-), BSM (bovine submaxillary mucin), LDN (GalNAcβ1-4GlcNAcβ1-), Forssman disaccharide (GalNAcα1-3GalNAcβ1-), βGalNAc (GalNAcβ1-) and demonstrates high affinity to glycans having αGalNAc at the non-reducing end. A list of glycans used in the glycan array is shown in Fig. 10.

### [Example 10] Flow Cytometry Analysis of Extracellular Vesicles using ASGR1-immobilized Beads

ASGR1-immobilized beads were produced by mixing 24 µg of the ASGR1-Fc fusion protein produced in Example 2 with 50 µl of Dynabeads Protein G (VERITAS Corporation) and binding the same for 30 minutes at 4 °C.

10 ml of the culture supernatant of 201B7 iPS cells cultured with mTeSR PLUS (VERITAS Corporation) was collected and concentrated to 1 ml with 100 kDa cut Amicon Ultra (Merck Ltd.). CaCl₂ was added such that the final concentration became 1 mM and the supernatant was mixed with the ASGR1-immobilized beads and incubated overnight at 4 °C while being mixed. After washing, 10 µg/ml of AF 647 human anti-CD63 (CD63 antibody, BD Biosciences) or a control antibody (isotype antibody) was reacted with the mixture and analysis with flow cytometry was performed.

As shown in Figure 11, the CD63 antibody ("CD63" in the figure) demonstrated significantly higher fluorescence intensity as compared with the control antibody ("Isotype" in the figure). It was understood that the extracellular vesicles can be analyzed with flow cytometry using the ASGR1-immobilized beads. Meanwhile, no reactivity was found when the culture supernatant was reacted with Protein G beads on which ASGR1-Fc was not immobilized ("Beads" in the figure) and detection was carried out with AF 647 human anti-CD63.

### [Example 11] Separation of HEK293T Cell-derived Extracellular Vesicles with ASGR1

ASGR1-immobilized beads were prepared from the culture supernatant of HEK293T cells with the same method as in Example 6 and extracellular vesicles were separated with the TIM4 method and the ultracentrifugation method.

The results of observation of the separated extracellular vesicles under transmission electron microscopy are shown in Figure 12.

The extracellular vesicles separated with ASGR1-immobilized beads were vesicles having a lipid bilayer and a particle diameter of about 200-300 nm.

### [Example 12] Proteomic Analysis in Extracellular Vesicles Separated with Different Separation Methods

ASGR1-immobilized beads were prepared from the culture supernatant of HEK293T cells with the same method as in Example 6 and extracellular vesicles were separated with the TIM4 method and the ultracentrifugation method.

Volumes equivalent to 1.5 µg in terms of total protein mass were respectively taken from suspensions of extracellular vesicles and subjected to acetone precipitation treatment, and the precipitates were dissolved in a solubilization buffer. A tryptic hydrolysis process was performed on the lysates to obtain peptide mixtures. MS/MS data obtained by liquid chromatography-tandem mass spectrometry (LC-MS/MS) of the peptide mixtures was collated with a human amino acid sequence database. Both peptide identification and peptide detection intensity information were combined and peptide detection intensity was integrated for each protein (protein measurement value) (Fig. 13).

The data obtained as described above was analyzed with the FunRich software (http://www.funrich.org/) to analyze the percentages of proteins associated with each cellular component (Fig. 14).

Furthermore, in the peptide mixtures obtained as described above, the expression intensity of the exosome markers described in the MISEV (Minimal Information for Studies of Extracellular Vesicles) guidelines (*MISEV2023*) were analyzed and shown in heatmaps using the software R (Fig. 15).

As shown in Figure 13, 1630 types of protein were detected in common with all three separation methods. There were a total of 1789 types of protein detected with the ASGR1-immobilized beads, a total of 2102 types of protein detected with the TIM4 method, and 2109 types of protein detected with the ultracentrifugation method. Further, 59 types of protein were detected only with the ASGR1-immobilized beads, 48 types of protein were detected only with the TIM4 method, and 25 types of protein were detected only with the ultracentrifugation method.

Based on the results of the present example, it was shown that extracellular vesicles having different types of protein can be separated than with conventional methods by separating using ASGR1.

As shown in Figure 14, exosome-associated proteins were detected in 49.80% of the extracellular vesicles separated with the ASGR1-immobilized beads, in 47.98% of those separated with the TIM4 method, and in 47.34% of those separated with the ultracentrifugation method.

Based on the results of the present example, it was shown that exosomes can be separated more selectively (at a higher purity) than with conventional methods by separating using ASGR1.

As shown in Figure 15, it was shown that the most exosome markers were included in the extracellular vesicles separated with the ASGR1-immobilized beads as compared with the TIM4 method and the ultracentrifugation method.

### [Example 13] Flow Cytometry Analysis using Exosome Marker Antibodies in HEK293T Cell-derived Extracellular Vesicles

Culture supernatant obtained by culturing HEK293T cells was incubated with ASGR1-immobilized beads or TIM4-immobilized beads (MagCapture^{™} Exosome Isolation Kit PS, FUJIFILM Wako Pure Chemical Corporation) overnight at 4 °C.

The beads were subsequently each suspended in wash buffer (Tris-HCl; NaCl; pH 7.4) to which 1 mM of CaCl₂ had been added and incubated with 10 µg/ml of a labeled isotype control antibody (clone number: A-1, MC2B-TF2, Cosmo Bio Co., Ltd) or an exosome marker (CD9, CD63, or CD81) antibody (clone number: 12A12, SHI-EXO-M01, -M02, and -M03 (maker: CAC), Cosmo Bio Co., Ltd).

Flow cytometry data was analyzed with CytoFLEX (Beckman Coulter, Inc.) and is shown in Fig. 16.

As shown in Fig. 16, the ASGR1-immobilized bead group and the TIM4-immobilized bead group differed in the detection pattern of exosomes positive for the exosome markers CD9, CD63, and CD81.

It was understood from the results of the present example that the expression pattern of each exosome marker in the obtained exosomes differed, that is, different groups of exosomes were separated between the case where the exosomes obtained from the HEK293T cells were separated with the ASGR1-immobilized beads and the case where the exosomes were separated with the TIM4 method.

### [Example 14] Flow Cytometry Analysis using Exosome Marker Antibodies in iPS Cell-derived Extracellular Vesicles

The culture supernatant of 201B7 iPS cell (human iPS cell line) was incubated with ASGR1-immobilized beads or TIM4-immobilized beads (MagCapture^{™} Exosome Isolation Kit PS, FUJIFILM Wako Pure Chemical Corporation) overnight at 4 °C.

The beads were subsequently each suspended in wash buffer (Tris-HCl; NaCl; pH 7.4) to which 1 mM of CaCl₂ had been added and incubated with 10 µg/ml of a labeled isotype control antibody (clone number: A-1, MC2B-TF2, Cosmo Bio Co., Ltd) or an exosome marker (CD9, CD63, or CD81) antibody (clone number: 12A12, SHI-EXO-M01, -M02, and -M03 (maker: CAC), Cosmo Bio Co., Ltd).

Flow cytometry data was analyzed with CytoFLEX (Beckman Coulter, Inc.) and is shown in Fig. 17.

As shown in Fig. 17, the ASGR1-immobilized bead group and the TIM4-immobilized bead group differed in the detection pattern of exosomes positive for the exosome markers CD9, CD63, and CD81.

It was understood from the results of the present example that the expression pattern of each exosome marker in the obtained exosomes differed, that is, different groups of exosomes were separated between the case where the exosomes obtained from the iPS cells were separated with the ASGR1-immobilized beads and the case where the exosomes were separated with the TIM4 method.

### [Example 15] ELISA Analysis with a CD63 Antibody in Extracellular Vesicles Derived from Different Cells

HEK293T cells were cultured with RPMI1640, iPS cells were cultured with mTeSR1 (STEMCELL Technologies), and adipose-derived mesenchymal stem cells (ADSC, Lot No. 2118, Life Technologies) and bone marrow-derived mesenchymal stem cells (Yub622, RIKEN Cell Bank) were cultured with MesenPRO RS^{™} Medium (Catalog No.: 12746-012, Gibco) (2 mM L-glutamine and 1% penicillin-streptomycin added).

100 µl of 2 µg/ml biotinylated ASGR1-Fc was added to avidin immobilization plates (BS-X7603, Sumitomo Bakelite Co., Ltd.) and immobilized by incubating for 1 hour at room temperature.

After washing, 100 µl/well of the culture supernatant of the HEK293T cells, 201B7 iPS cells, adipose-derived mesenchymal stem cells, or bone marrow-derived mesenchymal stem cells was added and reacted for 2 hours at room temperature. At this time, the culture supernatants were diluted in three steps (by a factor of 2 (0.5), by a factor of 4 (0.25), and by a factor of 8 (0.125)) as shown on the horizontal axes of Fig. 18A-D. The culture supernatants were washed and removed and a peroxidase-labeled anti-CD63 antibody (SHI-EXO-M02 (Cosmo Bio Co., Ltd.) labeled with a peroxidase (LK11, DOJINDO LABORATORIES) (0.5 µg/ml, 100 µl) were added and reacted for 2 hours at room temperature. Lastly, 100 µl/well of TMB solution (FUJIFILM Wako Pure Chemical Corporation) was added and after being reacted for 30 min. at room temperature, the coloring reaction was stopped with 1 N HCl and measured at OD 450/620 nm.

As shown in Figure 18, the presence of the exosome marker CD63 was detected in the culture supernatant of all of the cells. This indicates that CD63-positive exosomes were separated from all the cell culture supernatants with ASGR1.

Note that the CD63-positive exosomes did not depend on culture supernatant dilution for the HEK293T cells, adipose-derived mesenchymal stem cells, or bone marrow-derived mesenchymal stem cells. This indicates that these cell supernatants included, at high concentrations, CD63-positive exosomes that the ASGR1 immobilized on the plates was saturated with.

### INDUSTRIAL APPLICABILITY

The method of separation of extracellular vesicles and the kit for separating extracellular vesicles according to the present disclosure are capable of separating extracellular vesicles from a sample including the extracellular vesicles, and therefore can be suitably used for diagnosis, treatment, etc. using the extracellular vesicles and have industrial applicability.

## Claims

1. A method for separating extracellular vesicles in a sample, comprising:
contacting the extracellular vesicles in the sample with a C-type lectin; and
separating the extracellular vesicles bound to the C-type lectin from the C-type lectin.

2. The separation method according to claim 1, comprising:
contacting the extracellular vesicles in the sample with the C-type lectin in the presence of calcium ions; and
separating the extracellular vesicles bound to the C-type lectin from the C-type lectin in the absence of calcium ions.

3. The separation method according to claim 1 or 2, wherein the C-type lectin is ASGR1, DCSIGN, and/or CLEC1B.

4. The separation method according to claim 1 or 2, wherein the C-type lectin is ASGR1.

5. A kit for separating extracellular vesicles from a sample comprising extracellular vesicles, wherein the kit comprises a C-type lectin.

6. A kit for detecting extracellular vesicles, comprising a C-type lectin and an antibody that specifically binds to the extracellular vesicles.

7. The kit according to claim 5 or 6, wherein the C-type lectin is ASGR1, DCSIGN, and/or CLEC1B.

8. The kit according to claim 5 or 6, wherein the C-type lectin is ASGR1.
